# EUROPEAN PATENT APPLICATION

(11) **EP 2 601 985 A1**
(43) Date of publication of application: **12.06.2013**
(21) Application number: 11814585.3
(22) Date of filing: 29.07.2011
(51) Int. Cl.: A61M 1/14

(54) **BLOOD PURIFYING DEVICE, AND METHOD FOR INSPECTING FOR LIQUID LEAKAGE THEREIN**

(30) Priority: 05.08.2010 JP 2010176628
(71) Applicant: Nikkiso Co., Ltd., Tokyo 150-6022 (JP)
(72) Inventor: SAKAMOTO, Kazuya, Makinohara-shi Shizuoka 421-0496 (JP); FURUHASHI, Tomohiro, Makinohara-shi Shizuoka 421-0496 (JP)
(74) Representative: von Hirschhausen, Helge
(86) International application number: PCT/JP2011/067508
(87) International publication number: WO 2012/017959

(57) **Abstract**

An object of the present invention is to provide a blood purification apparatus and the method for inspecting liquid leakage therefrom which can perform a sufficient liquid leakage inspection over a whole region of the blood circuit. According to the present invention, there is provided a blood purification apparatus comprising a blood circuit including an arterial blood circuit arranged thereon a blood pump and a venous blood circuit for extracorporeally circulating blood of a patient by the blood pump; a blood purification instrument for purifying the blood of a patient extracorporeally circulated through the blood circuit adapted to be connected with a base end of the arterial blood circuit and a base end of the venous blood circuit of the blood circuit; a dialysate introducing line for introducing dialysate into the blood purifying instrument; and a dialysate discharging line for discharging the dialysate from the blood purifying instrument characterized in that the blood purification apparatus further comprises a pressure varying means for varying pressure in a closed circuit under a condition in which the blood circuit is formed as a closed circuit of a sealed condition with connecting the tip end of the arterial blood circuit and the tip end of the venous blood circuit; and a liquid leakage detecting means for detecting liquid leakage in the blood circuit in accordance with pressure variation generated by the pressure varying means.

## Description

### Field of the Invention

The present invention relates to a blood purification apparatus having a blood purification instrument (i.e. dialyzer) for purifying blood of a patient extracorporeally circulating in a blood circuit and a method for inspecting liquid leakage of the blood purification apparatus.

### Description of Background Art

In general, in the blood purification apparatus used in the dialysis treatment, a dialysate introducing line and a dialysate discharging line respectively for supplying the dialysate to the dialyzer and for discharging the dialysate containing waste materials produced by dialysis from the dialyzer are connected to the dialyzer connected to the blood circuit. Tip ends of the dialysate introducing line and the dialysate discharging line are connected respectively to a dialysate introducing port and a dialysate discharging port and base ends of them are connected respectively to a dialysate supplying apparatus and a dialysate discharging apparatus.

Technology of automatically detecting liquid leakage by applying a positive pressure to the blood circuit as well as dialysate lines of a main body side of the hemodialysis apparatus including the dialysate introducing line and the dialysate discharging line is disclosed for example in Patent Document 1 below. The liquid leakage detecting technology of this prior art is formed to detect the liquid leakage by closing a venous blood circuit at a portion near its tip end using an electromagnetic valve etc., by driving a blood pump arranged in a arterial blood circuit to apply the positive pressure thereto, and by detecting a pressure drop caused thereafter.

### Document of the prior art

### Patent Document

[Patent Document 1] Japanese Laid-open Patent Publication No. 253550/1999

### Disclosure of the Invention

### Problems to be solved by the Invention

However, according to the blood purification apparatus and the method for inspecting liquid leakage therefrom, since it is formed so that the liquid leakage is detected by closing a venous blood circuit at a portion near its tip end using an electromagnetic valve etc. and the positive pressure is applied by driving a blood pump arranged in a arterial blood circuit, it is impossible to perform the liquid leakage inspection of a portion of the venous blood circuit positioned nearer to its tip end than the portion closed by the electromagnetic valve as well as a portion of the arterial blood circuit positioned nearer to its tip end than the blood pump. In addition, since the liquid leakage inspection of the prior art is performed only by applying the positive pressure and thus the liquid leakage inspection due to application of the negative pressure is not performed, sufficient liquid leakage inspection cannot be achieved by the prior art.

It is, therefore, an object of the present invention to provide a blood purification apparatus and the method for inspecting liquid leakage therefrom which can perform a sufficient liquid leakage inspection over a whole region of the blood circuit.

### Means for solving the Problems

For achieving the object of the present invention, there is provided according to the present invention of claim 1 a blood purification apparatus comprising a blood circuit including an arterial blood circuit arranged thereon a blood pump and a venous blood circuit for extracorporeally circulating blood of a patient by the blood pump; a blood purification instrument for purifying the blood of a patient extracorporeally circulated through the blood circuit adapted to be connected with a base end of the arterial blood circuit and a base end of the venous blood circuit of the blood circuit; a dialysate introducing line for introducing dialysate into the blood purifying instrument; and a dialysate discharging line for discharging the dialysate from the blood purifying instrument characterized in that the blood purification apparatus further comprises a pressure varying means for varying pressure in a closed circuit under a condition in which the blood circuit is formed as a closed circuit of a sealed condition with connecting the tip end of the arterial blood circuit and the tip end of the venous blood circuit; and a liquid leakage detecting means arranged in the closed circuit for detecting liquid leakage in the blood circuit in accordance with pressure variation generated by the pressure varying means.

The invention of claim 2 is a blood purification apparatus of claim 1 characterized in that the pressure varying means varies liquid pressure in the closed circuit under a condition in which the blood circuit is filled with priming liquid.

The invention of claim 3 is a blood purification apparatus of claim 1 or 2 characterized in that the pressure varying means comprises a liquid supplying means for increasing pressure in the closed circuit by applying a positive pressure to the closed circuit with introducing liquid into the closed circuit from the outside thereof.

The invention of claim 4 is a blood purification apparatus of claim 3 characterized in that the blood purification apparatus further comprises a discharging line for discharging liquid or gas in the closed circuit to the outside thereof, and that the liquid leakage in the blood circuit is detected by the liquid leakage detecting means during a pressurizing step by alternately performing the pressurizing step and a priming step, in the pressurizing step the positive pressure being applied to the closed circuit by introducing liquid into the closed circuit from the outside thereof by the liquid supplying means with keeping a closed condition of the discharging line, and in the priming step liquid being introduced into the closed circuit from the outside thereof by the liquid supplying means with keeping a opened condition of the discharging line and then liquid or gas in the closed circuit being discharged from the discharging line.

The invention of claim 5 is a blood purification apparatus of claim 3 or 4 characterized in that the liquid leakage detecting means comprises a pressure detecting means for detecting pressure in the closed circuit after a positive pressure has been applied to the closed circuit; and a decision means for deciding existence of liquid leakage in the blood circuit on the basis of the pressure detected by the pressure detecting means.

The invention of claim 6 is a blood purification apparatus of claim 1 or 2 characterized in that the pressure varying means comprises a liquid supplying means for decreasing pressure in the closed circuit by applying a negative pressure to the closed circuit with extracting liquid from the closed circuit to the outside thereof.

The invention of claim 7 is a blood purification apparatus of claim 6 characterized in that the liquid leakage detecting means comprises a pressure detecting means for detecting pressure in the closed circuit after a negative pressure has been applied to the closed circuit; and a decision means for deciding existence of liquid leakage in the blood circuit on the basis of the detected pressure by the pressure detecting means.

The invention of claim 8 is a blood purification apparatus of claim 5 or 7 characterized in that the pressure detecting means comprises one arranged in the closed circuit and the other one arranged in the dialysate introducing line or the dialysate discharging line, and that the decision means decides existence of liquid leakage in the blood circuit by comparing a pressure detected by the pressure detecting means arranged in the closed circuit with a pressure detected by the pressure detecting means arranged in the dialysate introducing line or the dialysate discharging line.

The invention of claim 9 is a blood purification apparatus of claim 6 characterized in that the liquid leakage detecting means comprises an air bubble detecting means for detecting bubbles which would be generated in case of liquid leakage in the blood circuit when the negative pressure is applied to the closed circuit; and a decision means for deciding existence of liquid leakage on the basis of a fact whether the air bubbles are detected.

The invention of claim 10 is a blood purification apparatus of any one of claims 3∼9 characterized in that the liquid supplying means comprises a dialysate pump for introducing the dialysate to the blood purifying instrument, a ultrafiltration pump for performing ultrafiltration against blood circulating extracorporeally through the blood circuit, or a substitution infusing pump for introducing substitution to the blood circuit.

The invention of claim 11 is a blood purification apparatus of claim 10 that the liquid supplying means is a pump being able to perform a normal rotation and a reverse rotation, and that either the application of positive pressure or negative pressure to the closed circuit can be achieved by selectively performing the normal rotation or the reverse rotation.

The invention of claim 12 is a blood purification apparatus of claim 1 or 2 that the pressure varying means is able to increase pressure in the closed circuit by applying a positive pressure to the closed circuit with introducing liquid into the closed circuit from the outside thereof and also able to decrease pressure in the closed circuit by applying a negative pressure to the closed circuit with extracting liquid from the closed circuit to the outside thereof, and that the liquid leakage detecting means is able to detect liquid leakage both in cases of application of the positive pressure and negative pressure.

The invention of claim 13 is a blood purification apparatus of claim 12 characterized in that the positive pressure is applied to the closed circuit after the negative pressure has been applied to the closed circuit.

The invention of claim 14 is a blood purification apparatus of any one of claims 1∼13 characterized in that the pressure varying means varies pressure in the closed circuit by introducing liquid to the closed circuit through the dialysate introducing line or by discharging liquid from the closed circuit to the dialysate discharging line, and that the liquid leakage detecting means is able to detect liquid leakage in the dialysate introducing line or the dialysate discharging line in addition to liquid leakage in the closed circuit.

According to the present invention of claim 15, there is also provided a method for inspecting liquid leakage of a blood purification apparatus comprising a blood circuit including an arterial blood circuit arranged thereon a blood pump and a venous blood circuit for extracorporeally circulating blood of a patient by the blood pump; a blood purification instrument for purifying the blood of a patient extracorporeally circulated through the blood circuit adapted to be connected with a base end of the arterial blood circuit and a base end of the venous blood circuit of the blood circuit; a dialysate introducing line for introducing dialysate to the blood purifying instrument; and a dialysate discharging line for discharging the dialysate from the blood purifying instrument characterized in that the method comprises a pressure varying step for varying pressure in a closed circuit under a condition in which the blood circuit is formed as a closed circuit of a sealed condition with connecting the tip end of the arterial blood circuit and the tip end of the venous blood circuit; and a liquid leakage detecting step for detecting liquid leakage in the blood circuit in accordance with pressure variation generated by the pressure varying step.

The invention of claim 16 is a method for inspecting liquid leakage of a blood purification apparatus of claim 15 characterized in that in the pressure varying step means, the liquid pressure in the closed circuit is varied under a condition in which the blood circuit is filled with priming liquid.

The invention of claim 17 is a method for inspecting liquid leakage of a blood purification apparatus of claim 16 characterized in that in the pressure varying step, the pressure in the closed circuit is increased by applying a positive pressure to the closed circuit with introducing liquid into the closed circuit from the outside thereof.

The invention of claim 18 is a method for inspecting liquid leakage of a blood purification apparatus of claim 17 characterized in that the blood purification apparatus further comprises a discharging line for discharging liquid or gas in the closed circuit to the outside thereof, and that the liquid leakage detecting step is performed during a pressurizing step by alternately performing the pressurizing step and a priming step, in the pressurizing step the positive pressure being applied to the closed circuit by introducing liquid into the closed circuit from the outside thereof by the liquid supplying means with keeping a closed condition of the discharging line, and in the priming step liquid being introduced into the closed circuit from the outside thereof by the liquid supplying means with keeping a opened condition of the discharging line and then liquid or gas in the closed circuit being discharged from the discharging line.

The invention of claim 19 is a method for inspecting liquid leakage of a blood purification apparatus of claim 18 characterized in that the liquid leakage detecting step comprises a pressure detecting step for detecting pressure in the closed circuit after a positive pressure has been applied to the closed circuit; and a decision step for deciding existence of liquid leakage in the blood circuit on the basis of the pressure detected in the pressure detecting step.

The invention of claim 20 is a method for inspecting liquid leakage of a blood purification apparatus of claim 15 or 16 characterized in that in the pressure varying step, the pressure in the closed circuit is decreased by applying a negative pressure to the closed circuit with extracting liquid from the closed circuit to the outside thereof.

The invention of claim 21 is a method for inspecting liquid leakage of a blood purification apparatus of claim 20 characterized in that the liquid leakage detecting step comprises a pressure detecting step for detecting pressure in the closed circuit after a negative pressure has been applied to the closed circuit; and a decision step for deciding existence of liquid leakage in the blood circuit on the basis of pressure detected in the pressure detecting step.

The invention of claim 22 is a method for inspecting liquid leakage of a blood purification apparatus of claim 19 or 21 characterized in that in the pressure detecting step, pressures in the closed circuit and in the dialysate introducing line or the dialysate discharging line are detected respectively, and that the liquid leakage in the blood circuit is decided by comparing a pressure detected in the closed circuit with a pressure detected in the dialysate introducing line or the dialysate discharging line.

The invention of claim 23 is a method for inspecting liquid leakage of a blood purification apparatus of claim 20 characterized in that the liquid leakage detecting step comprises an air bubble detecting step for detecting bubbles which would be generated in case of liquid leakage in the blood circuit when the negative pressure is applied to the closed circuit; and a decision step for deciding existence of liquid leakage on the basis of a fact whether the air bubbles are detected.

The invention of claim 24 is a method for inspecting liquid leakage of a blood purification apparatus of any one of claims 17∼23 characterized in that the introduction or discharge of liquid to or from the closed circuit in the pressure varying step is performed by a dialysate pump for introducing the dialysate to the blood purifying instrument, a ultrafiltration pump for performing ultrafiltration against blood circulating extracorporeally through the blood circuit, or a substitution infusing pump for introducing substitution to the blood circuit.

The invention of claim 25 is a method for inspecting liquid leakage of a blood purification apparatus of claim 24 characterized in that the introduction or discharge of liquid to or from the closed circuit in the pressure varying step is achieved by a pump being able to perform a normal rotation and a reverse rotation, and that either the application of positive pressure or negative pressure to the closed circuit can be achieved by selectively performing the normal rotation or the reverse rotation.

The invention of claim 26 is a method for inspecting liquid leakage of a blood purification apparatus of claim 15 or 16 characterized in that in the pressure varying step, the pressure in the closed circuit is increased by applying a positive pressure to the closed circuit with introducing liquid into the closed circuit from the outside thereof and also decreased by applying a negative pressure to the closed circuit with extracting liquid from the closed circuit to the outside thereof, and that the liquid leakage detection is performed both in cases of application of the positive pressure and negative pressure.

The invention of claim 27 is a method for inspecting liquid leakage of a blood purification apparatus of claim 26 characterized in that the positive pressure is applied to the closed circuit after the negative pressure has been applied to the closed circuit.

The invention of claim 28 is a method for inspecting liquid leakage of a blood purification apparatus of any one of claims 15∼27 characterized in that in the pressure varying step, the pressure in the closed circuit is varied by introducing liquid to the closed circuit through the dialysate introducing line or by discharging liquid from the closed circuit to the dialysate discharging line, and that in the liquid leakage detecting step, the liquid leakage in the dialysate introducing line or the dialysate discharging line is detected in addition to liquid leakage in the closed circuit.

### Effects of the Invention

According to the present inventions of claims 1 and 15, since the liquid leakage in the blood circuit is detected by varying pressure in a closed circuit formed by connecting a tip end of the arterial blood circuit and a tip end of the venous blood circuit, it is possible to perform a sufficient liquid leakage inspection over a whole region of the blood circuit.

According to the present inventions of claims 2 and 16, since the liquid leakage in the blood circuit is detected by varying the liquid pressure in the closed circuit under a condition in which the blood circuit is filled with priming liquid, it is possible to perform a sufficient liquid leakage inspection over a whole region of the blood circuit.

According to the present inventions of claims 3 and 17, since the pressure variation in the closed circuit is performed by increasing the pressure with applying a positive pressure to the closed circuit by introducing liquid into the closed circuit from the outside thereof, it is possible to prevent suck of air into the blood circuit in case of the liquid leakage and thus to smoothly and properly perform the blood purification treatment.

According to the present inventions of claims 4 and 18, since the blood purification apparatus further comprises a discharging line for discharging liquid or gas in the closed circuit to the outside thereof; and the liquid leakage in the blood circuit is detected during a pressurizing step by alternately performing the pressurizing step and a priming step, in the pressurizing step the positive pressure being applied to the closed circuit by introducing liquid into the closed circuit from the outside thereof by the liquid supplying means with keeping a closed condition of the discharging line, and in the priming step liquid being introduced into the closed circuit from the outside thereof by the liquid supplying means with keeping a opened condition of the discharging line and then liquid or gas in the closed circuit being discharged from the discharging line, it is possible to perform the detection of liquid leakage during a process in which the priming (discharge of air bubbles and filling of priming liquid) is performed.

According to the present inventions of claims 5 and 19, since the liquid leakage detection is performed by detecting pressure in the closed circuit after a positive pressure has been applied to the closed circuit, and by deciding existence of liquid leakage in the blood circuit on the basis of the detected pressure, it is possible to detect the pressure when the positive pressure is applied to the closed circuit for example by substituting a venous pressure sensor usually connected to an air trap chamber connected to the venous blood circuit for a positive pressure detecting sensor and thus to reduce the manufacturing cost of the blood purification apparatus.

According to the present inventions of claims 6 and 20, since the pressure varying step is performed by applying a negative pressure to the closed circuit with extracting liquid from the closed circuit to the outside thereof to decrease the pressure in the closed circuit, it is possible to achieve the liquid leakage test due to the negative pressure of a portion to which the negative pressure is applied (e.g. a portion nearer to the tip end side of the arterial blood circuit than the blood pump) during the blood purification treatment and also possible to achieve the liquid leakage test proper for actions applied during the blood purification treatment.

According to the present inventions of claims 7 and 21, since the liquid leakage detection is performed by detecting pressure in the closed circuit after a negative pressure has been applied to the closed circuit, and by deciding existence of liquid leakage in the blood circuit on the basis of the detected pressure, it is possible to detect the pressure when the negative pressure is applied to the closed circuit for example by substituting a venous pressure sensor usually connected to an air trap chamber connected to the venous blood circuit for a negative pressure detecting sensor and thus to reduce the manufacturing cost of the blood purification apparatus.

According to the present inventions of claims 8 and 22, since the pressure detection is performed respectively in the closed circuit, the dialysate introducing line or the dialysate discharging line and existence of the liquid leakage is decided by comparing a pressure detected in the closed circuit with a pressure detected in the dialysate introducing line or the dialysate discharging line, it is possible to further improve the accuracy of decision of the liquid leakage.

According to the present inventions of claims 9 and 23, since the detection of liquid leakage is performed by detecting bubbles generated in a case of liquid leakage in the blood circuit when the negative pressure is applied to the closed circuit, it is possible to detect the bubbles even when the negative pressure is applied to the closed circuit by substituting a bubble detecting sensor etc. usually connected to the arterial blood circuit at a position nearer to the tip end than the air trap chamber for the bubble detecting sensor and thus possible to reduce the manufacturing cost of the blood purification apparatus. In addition, since bubbles are generated in a case of liquid leakage, the liquid leakage can be detected by visual observation.

According to the present inventions of claims 10 and 24, since the introduction and discharge of liquid to or from the closed circuit is performed by using the dialysate pump for introducing dialysate to the blood purification instrument, the ultrafiltration pump for performing ultrafiltration against blood extracorporeally circulating through the blood circuit or the substitution infusing pump, it is possible to substitute pumps used in the blood purification treatment for these pumps and thus to also reduce the manufacturing cost of the blood purification apparatus.

According to the present inventions of claims 11 and 25, since the introduction or discharge of liquid to or from the closed circuit in the pressure varying step is achieved by a pump being able to perform a normal rotation and a reverse rotation, and either the application of positive pressure or negative pressure to the closed circuit can be achieved by selectively performing the normal rotation or the reverse rotation, it is possible to arbitrarily and easily perform the application of positive pressure or negative pressure.

According to the present inventions of claims 12 and 26, since in the pressure varying step of the closed circuit, the pressure in the closed circuit is increased by applying a positive pressure to the closed circuit with introducing liquid into the closed circuit from the outside thereof and also decreased by applying a negative pressure to the closed circuit with extracting liquid from the closed circuit to the outside thereof, and thus the liquid leakage detection is performed both in cases of application of the positive pressure and negative pressure, it is possible to perform a proper and sufficient liquid leakage inspection.

According to the present inventions of claims 13 and 27, since the positive pressure is applied to the closed circuit after the negative pressure has been applied to the closed circuit, it is possible to prevent suck of air into the blood circuit through both tip ends of the arterial blood circuit and the venous blood circuit and thus to smoothly perform the blood purification treatment thereafter.

According to the present inventions of claims 14 and 28, since the liquid leakage in the dialysate introducing line or the dialysate discharging line can be detected in addition to liquid leakage in the closed circuit, it is possible to further improve the reliability of the blood purification apparatus.

### Brief Description of the Drawings

[Fig. 1] A schematic view showing a hemodialysis apparatus (before formation of a closed circuit) of a first embodiment of the present invention;
[Fig. 2] A schematic view showing a condition of the positive pressure application to the closed circuit in the hemodialysis apparatus of the first embodiment of the present invention;
[Fig. 3] A schematic view showing a condition of the negative pressure application to the closed circuit in the blood purification apparatus of the first embodiment of the present invention;
[Fig. 4] A schematic view showing a condition of the positive pressure application to the closed circuit in the blood purification apparatus of the first embodiment of the present invention;
[Fig. 5] A schematic view showing a condition of the positive pressure application to the closed circuit in the hemodialysis apparatus of a second embodiment of the present invention;
[Fig. 6] A schematic view showing a condition of the negative pressure application to the closed circuit in the blood purification apparatus of the second embodiment of the present invention;
[Fig. 7] A schematic view showing a condition of the positive pressure application to the closed circuit in the blood purification apparatus of the second embodiment of the present invention;
[Fig. 8] A schematic view showing a condition of the positive pressure application to the closed circuit in the hemodialysis apparatus of a third embodiment of the present invention;
[Fig. 9] A schematic view showing a condition of the negative pressure application to the closed circuit in the blood purification apparatus of the third embodiment of the present invention;
[Fig. 10] A schematic view showing a condition of a pressure applying step in the blood purification apparatus of the fourth embodiment of the present invention;
[Fig. 11] A schematic view showing a condition of a liquid leakage detection in the blood purification apparatus of the fourth embodiment of the present invention;
[Fig. 12] A schematic view showing a condition of a priming step in the blood purification apparatus of the fourth embodiment of the present invention;
[Fig. 13] A schematic view showing a condition of a circulation step in the blood purification apparatus of the fourth embodiment of the present invention;
[Fig. 14] A schematic view showing an embodiment in which pressure detecting means are connected both to the closed circuit and the dialysate introducing line (an upstream side from the electromagnetic valve V3) of the blood purification apparatus of the present invention;
[Fig. 15] A schematic view showing an embodiment in which pressure detecting means are connected both to the closed circuit and the dialysate introducing line (a downstream side from the electromagnetic valve V3) of the blood purification apparatus of the present invention;
[Fig. 16] A schematic view showing an embodiment in which pressure detecting means are connected both to the closed circuit and the dialysate introducing line (a downstream side from the electromagnetic valve V4) of the blood purification apparatus of the present invention;
[Fig. 17] A schematic view showing an embodiment in which pressure detecting means are connected both to the closed circuit and the dialysate introducing line (an upstream side from the electromagnetic valve V4) of the blood purification apparatus of the present invention;
[Fig. 18] A schematic view showing a condition of the positive or negative pressure application to the closed circuit in the hemodialysis apparatus of another embodiment of the present invention; and
[Fig. 19] A schematic view showing a condition of the positive pressure application to the closed circuit in the hemodialysis apparatus of a further embodiment of the present invention.

### Best Mode for carrying out the Invention

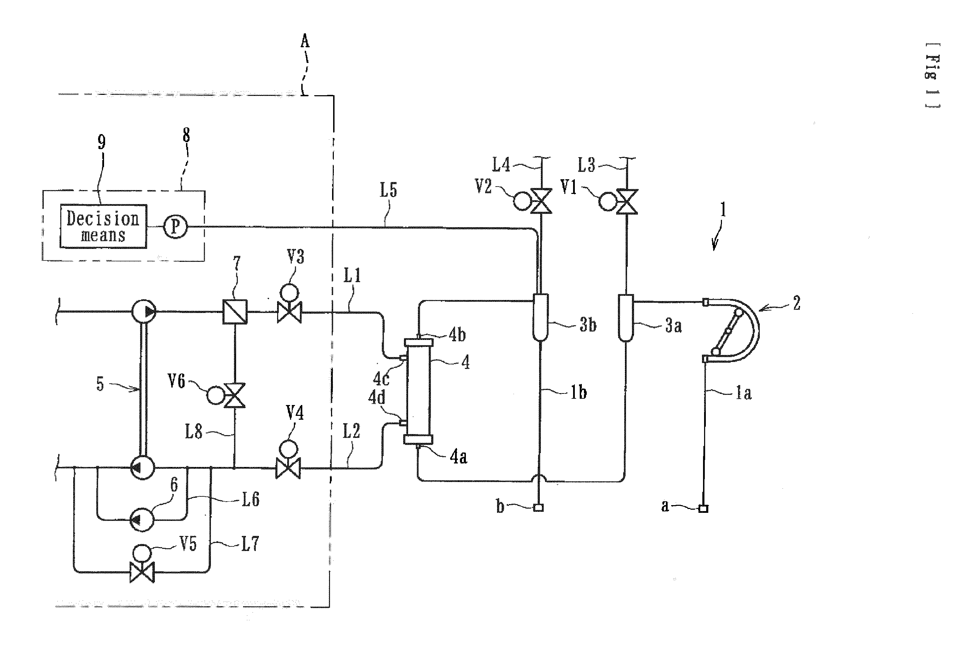

Preferable embodiments of the present invention will be hereinafter described with reference to the drawings.
A blood purification apparatus of a first embodiment of the present invention is adapted to be applied to a hemodialysis apparatus and mainly comprises, as shown in Figs. 1∼4, a blood circuit 1 including an arterial blood circuit 1a and a venous blood circuit 1b, a dialyzer 4 functioning as a blood purification instrument, a dialysate introducing line L1 and a dialysate discharging line L2, a duplex pump 5 and a ultrafiltration pump 6 functioning as liquid supplying means forming a pressure varying means, and a liquid leakage detecting means 8. The duplex pump 5 also functions as a dialysate pump (pump for introducing the dialysate to the dialyzer 4). A character "A" in drawings denotes a main body of the hemodialysis apparatus of the present invention.

The dialyzer 4 is intended to purify extracorporeally circulating blood of a patient and contains therein membranes (not shown, hollow fiber membranes in the present invention, however including semi-permeable membranes and filtration membranes). The dialyzer 4 is provided with a blood introducing port 4a for introducing blood, a blood discharging port 4b, for discharging the introduced blood, a dialysate introducing port 4c for introducing dialysate to which a tip end of the dialysate introducing line L1 is connected, and a dialysate discharging port 4d for discharging the introduced dialysate to which a tip end of the dialysate discharging line L2 is connected. Thus, as well known in the art, the blood purification action can be performed by contacting the dialysate with the blood introduced through the blood introducing port 4a via the hollow fiber membranes.

The blood introducing port 4a is connected with a base end of the arterial blood circuit 1a on which a blood pump 2 and an arterial air trap chamber 3a is arranged and the blood discharging port 4b is connected with a base end of the venous blood circuit 1b on which a venous air trap chamber 3b is arranged. A connector "a" and a connector "b" arranged on the tip ends respectively of the arterial blood circuit 1a and the venous blood circuit 1b are adapted to be attached respectively with an arterial puncture needle and a venous puncture needle.

Thus, it is possible to extracorporeally circulate blood of a patient while puncturing the arterial puncture needle and the venous puncture needle into an artery and a vein of a patient and driving the blood pump 2. During the extracorporeal circulation of the blood of a patient, bubble removal is performed through the arterial air trap chamber 3a connected the arterial blood circuit 1a and the venous air trap chamber 3b connected to the venous blood circuit 1b. The blood pump 2 is formed by a peristaltic pump (peristaltic pump being able to feed blood from the arterial puncture needle toward the blood introducing port 4a of the dialyzer 4 with squeezing a flexible tube forming the arterial blood circuit 1a by normally rotating the pump).

Overflow lines L3, L4 are connected to top end portions (air layer) respectively of the arterial air trap chamber 3a and venous air trap chamber 3b and electromagnetic valves V1, V2 are also arranged respectively on the overflow lines L3, L4. Thus, it is possible to arbitrarily open and close the flow paths of the overflow lines L3, L4 by opening and closing the electromagnetic valves V1, V2. According to the present invention, it is possible to form a sealed condition of the blood circuit 1 by connecting the tip ends of the arterial blood circuit 1a and the venous blood circuit 1b and by closing the electromagnetic valves V1, V2.

In addition, a liquid pressure monitoring line L5 is extended from the top end portion (air layer) of the venous air trap chamber 3b and the tip end of the liquid pressure monitoring line L5 is connected to a venous pressure sensor P arranged within the main body "A" of the hemodialysis apparatus. The venous pressure sensor P is able to detect the liquid pressure in the blood circuit 1 (more particularly the venous blood circuit 1b) by detecting the pressure in the top portion (air layer) of the venous air trap chamber 3b. Thus, it is possible to continuously (in real time) detect the venous pressure during the blood purification treatment (hemodialysis treatment).

On the other hand, base ends of the dialysate introducing line L1 and the dialysate discharging line L2 are connected respectively to a dialysate supplying apparatus (dialysate supplying source, not shown) and a dialysate discharging apparatus (also not shown) and the duplex pump (liquid supplying means) 5 is arranged across the dialysate introducing line L1 and the dialysate discharging line L2. With operating the duplex pump 5, the prepared dialysate is supplied to the dialyzer 4 from the dialysate supplying apparatus through the dialysate introducing line L1 and the used dialysate is discharged from the dialyzer 4 and returned to the dialysate discharging apparatus through the dialysate discharging line L2.

Bypass line L6, L7 bypassing the duplex pump 5 are provided on the dialysate discharging line L2. A ultrafiltration pump (liquid supplying means) 6 for removing water content from blood of a patient flowing through the dialyzer 4 is arranged on the bypass line L6 and an electromagnetic valve V5 for opening and closing a flow path of the bypass line L7 is arranged thereon. In addition, an electromagnetic valve V4 for opening and closing a flow path of the dialysate discharging line L2 is arranged thereon at a position near the dialysate discharging port 4d of the dialyzer 4.

Furthermore, a filter 7 is arranged on the dialysate introducing line L1 and an electromagnetic valve V3 for opening and closing a flow path of the dialysate introducing line L1 is arranged thereon at a position near the dialysate introducing port 4c of the dialyzer 4. The filter 7 is intended to filtrate and purify the dialysate flowing through the dialysate introducing line L1 and comprises a primary chamber in which dialysate to be filtrated flows through a filtering membrane and a secondary chamber in which filtrated dialysate flows. In addition, a bypass line L8 extends from the filter 7 for bypassing dialysate to the dialysate discharging line L2 and an electromagnetic valve V6 for opening and closing a flow path of the bypass line L8 is arranged thereon.

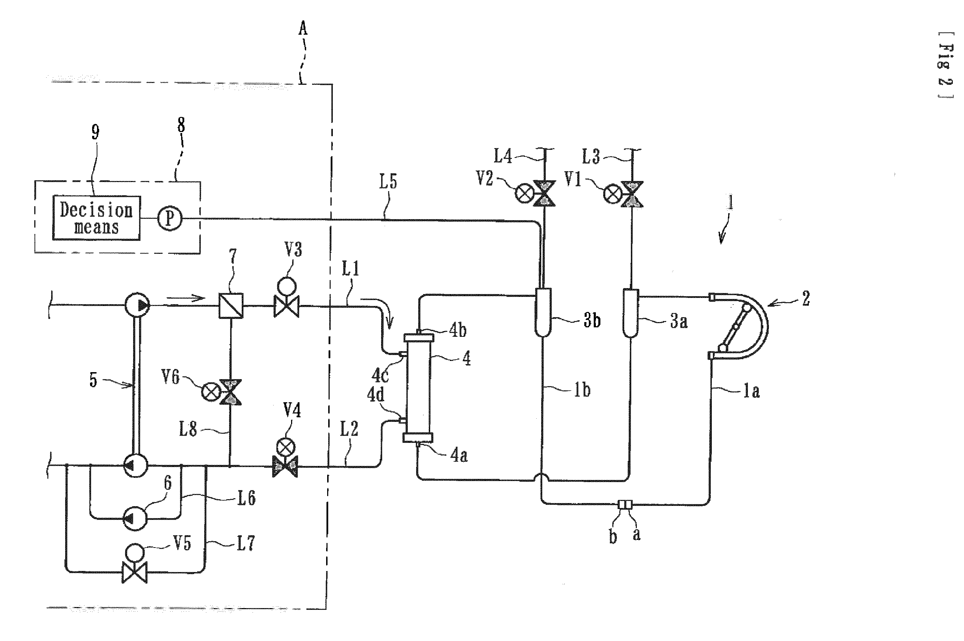

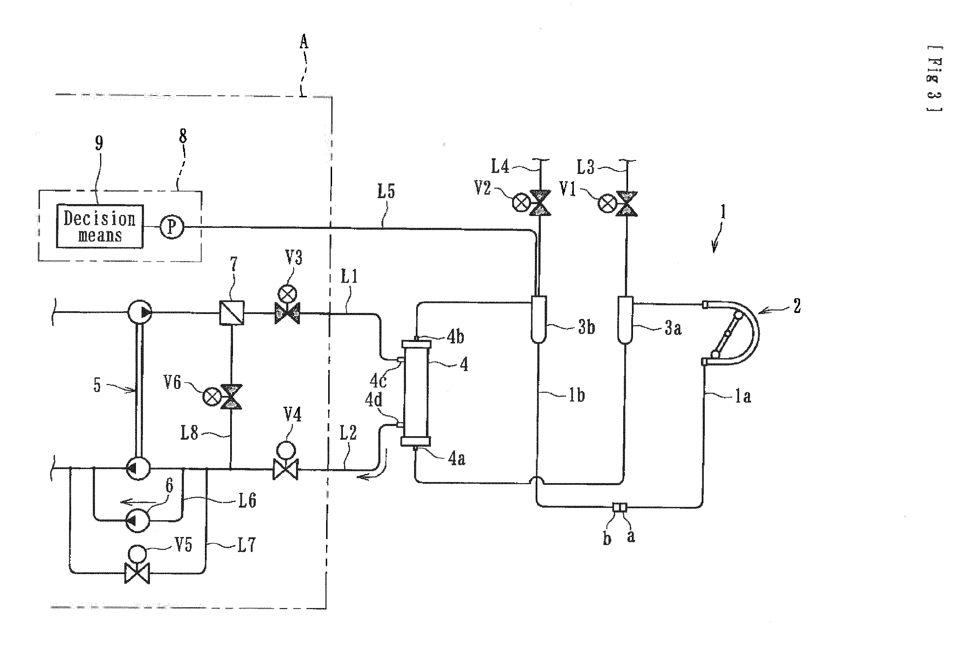

As shown in Figs. 2 and 3, the blood purification apparatus of this embodiment comprises a pressure varying means for varying pressure in a closed circuit under a condition in which the blood circuit 1 is formed as a closed circuit of a sealed condition with connecting the tip end (connector) "a" of the arterial blood circuit 1a and the tip end (connector) "b" of the venous blood circuit 1b and in which the blood circuit 1 is filled with priming liquid, and a liquid leakage detecting means 8 arranged in the closed circuit for detecting liquid leakage in the blood circuit 1 in accordance with pressure variation generated by the pressure varying means.

The pressure varying means of the present invention comprises the duplex pump (liquid supplying means) 5 for increasing liquid pressure in the closed circuit by applying a positive pressure to the closed circuit with introducing liquid (dialysate) into the closed circuit from the outside (dialysate introducing line L1) of the closed circuit, and the ultrafiltration pump (liquid supplying means) 6 for decreasing liquid pressure in the closed circuit by applying a negative pressure to the closed circuit with extracting liquid (priming solution (dialysate)) from the closed circuit to the outside (dialysate discharging line L2). That is, according to the present invention, the pressure varying means is constructed so that it is possible to increase the liquid pressure in the closed circuit by applying a positive pressure to the closed circuit with introducing liquid (dialysate) into the closed circuit from the outside thereof using the duplex pump 5, and also possible to decrease the liquid pressure in the closed circuit by applying a negative pressure to the closed circuit with extracting liquid (priming solution) from the closed circuit to the outside thereof using the ultrafiltration pump 6 and thus it is possible to perform the liquid leakage detection using the liquid leakage detecting means 8 in the application of both the positive pressure and the negative pressure.

As described above, it is possible to apply the positive pressure to the closed circuit by forming the closed circuit of sealed condition in the blood circuit 1 with closing the electromagnetic valves V1, V2 and accordingly the overflow lines L3, L4 and driving the duplex pump (liquid supplying means) 5 with closing the electromagnetic valves V4, V6 and opening other electromagnetic valves as shown in Fig. 2 after having performed the priming with filling the blood circuit 1 with priming solution (e.g. dialysate or physiological saline) under the connected condition of the tip end (connector "a") of the arterial blood circuit 1a and the tip end (connector "b") of the venous blood circuit 1b, and thus by introducing (i.e. inverse filtrating) the dialysate in the dialysate introducing line L1 (portion between the duplex pump 5 and the dialyzer 4) and the dialysate discharging line L2 (portion between the dialyzer 4 and the electromagnetic valve V4) from the flow path of dialysate to the blood flow path through the filtration membrane (hollow fiber membrane in the present embodiment) of the dialyzer 4.

On the contrary, it is possible to apply the negative pressure to the closed circuit by forming the closed circuit of sealed condition in the blood circuit 1 with closing the electromagnetic valves V1, V2 and accordingly the overflow lines L3, L4 and normally rotating the ultrafiltration pump (liquid supplying means) 6 with closing the electromagnetic valves V3, V6 and opening other electromagnetic valves as shown in Fig. 3 after the priming having been performed with filling the blood circuit 1 with priming solution (e.g. dialysate or physiological saline) under the connected condition of the tip end (connector "a") of the arterial blood circuit 1a and the tip end (connector "b") of the venous blood circuit 1b, and thus by extracting (i.e. normally filtrating) the priming solution in the closed circuit to the dialysate discharging line L2 through the filtration membrane (hollow fiber membrane in the present embodiment) of the dialyzer 4.

The liquid leakage detecting means 8 comprises the venous pressure sensor P and a decision means 9. As previously described, the venous pressure sensor (liquid pressure detecting means) P is able to continuously (in real time) detect the venous pressure during the blood purification treatment (hemodialysis treatment) and additionally able to continuously (in real time) detect the liquid pressure after the positive pressure having been applied to the closed circuit by the duplex pump 5 and after the negative pressure having been applied to the closed circuit by the ultrafiltration pump 6.

The decision means 9 is formed of e.g. a microcomputer etc. arranged within the main body "A" of the hemodialysis apparatus and electrically connected to the venous pressure sensor (liquid pressure detecting means) P to decide existence of liquid leakage in the blood circuit 1 on the basis of liquid pressure detected by the venous pressure detecting means P. That is, the existence of liquid leakage is decided by the decision means 9 in accordance with tendency after variation of the detected liquid pressure with continuously (in real time) detecting the tendency after variation of the liquid pressure caused by the pressure varying means using the venous pressure sensor P. There are following methods for deciding the liquid leakage using such a decision means 9.

Firstly, the pressure varying means applies a positive or negative pressure to the closed circuit and keeps a constant liquid pressure. Then, the venous pressure sensor (liquid pressure detecting means) P detects a liquid pressure having been varied until a predetermined period has elapsed from said constantly kept liquid pressure and finally decides whether the detected liquid pressure exceeds a predetermined threshold value (upper limit or lower limit value). When the detected liquid pressure exceeds the threshold, since it is supposed that it is impossible to keep the constant liquid pressure due to the liquid leakage, it can be decided that the liquid leakage would exist in any portion of the closed circuit. In another method for deciding the liquid leakage using such a decision means 9, the pressure varying means applies a positive or negative pressure to the closed circuit. Then, the venous pressure sensor (liquid pressure detecting means) P detects the liquid pressure and decides whether the detected liquid pressure is a predetermined liquid pressure. When the detected liquid pressure does not exhibit the predetermined liquid pressure, since it is supposed that the liquid pressure cannot reach the predetermined value due to the liquid leakage, it can be decided that the liquid leakage would exist in any portion of the closed circuit.

Then, the method for inspecting liquid leakage of a dialysis apparatus of the first embodiment will be described.
Firstly, the liquid pressure in the closed circuit is varied by the pressure varying means under a condition in which the blood circuit 1 is formed as a closed circuit of a sealed condition with connecting the tip end of the arterial blood circuit 1a and the tip end of the venous blood circuit 1b and under a condition in which the blood circuit 1 is filled with priming solution (pressure varying step). Then, the liquid leakage in the blood circuit 1 is detected by the liquid leakage detecting means in accordance with the variation of the liquid pressure in the pressure varying step (liquid leakage detecting step).

The liquid leakage detecting step comprises a pressure detecting step for detecting liquid pressure in the closed circuit after a positive pressure or a negative pressure has been applied to the closed circuit; and a decision step for deciding existence of liquid leakage in the blood circuit 1 on the basis of the liquid pressure detected in the pressure detecting step. In addition, in the pressure varying step, the liquid pressure in the closed circuit is increased by applying a positive pressure to the closed circuit by driving the duplex pump 5 with introducing liquid into the closed circuit from the outside thereof and also decreased by applying a negative pressure to the closed circuit by normally rotating the ultrafiltration pump 6 with extracting liquid from the closed circuit to the outside thereof. When the liquid leakage is detected in the liquid leakage detecting step, the fact is informed to an operator to urge him confirming the liquid leakage.

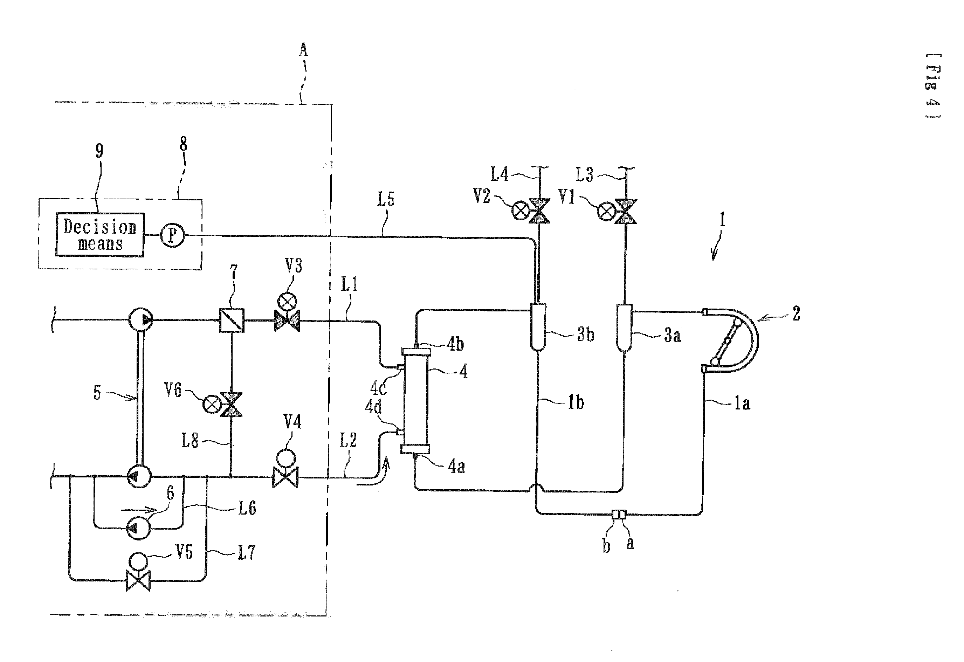

It is preferable that the ultrafiltration pump 6 functioning as the liquid supplying means is a pump being able to rotate both in normal and reverse directions so that either a positive pressure or a negative pressure can be applied to the closed circuit by selectively driving the ultrafiltration pump 6 in the normal direction or reverse direction. That is, as previously described, when the ultrafiltration pump 6 is normally rotated, it is possible to apply the negative pressure to the closed circuit. On the contrary, as shown in Fig. 4, when the ultrafiltration pump 6 is reversely rotated, the dialysate in the dialysate discharging line L2 is introduced to the flow path of blood from the flow path of dialysate of the dialyzer 4 through the filtration membrane (hollow fiber membrane in the present embodiment) (inverse filtration). Thus, the positive pressure can be applied to the closed circuit. According to such a construction, it is possible to easily and arbitrary apply the positive pressure and negative pressure to the closed circuit.

As described above, the pressure varying means and the pressure varying step can vary the liquid pressure in the closed circuit by introducing liquid to the closed circuit from the dialysate introducing line L1 or by extracting liquid from the closed circuit to the dialysate discharging line L2, and the liquid leakage detecting means and the liquid leakage detecting step can detect the liquid leakage of the dialysate introducing line L1 or the dialysate discharging line L2 (strictly, liquid leakage in a positive pressure applying region of the dialysate introducing line L1 or in a negative pressure applying region of the dialysate discharging line L2) in addition to the closed circuit. Accordingly, since it is possible to detect liquid leakage of the dialysate introducing line L1 or the dialysate discharging line L2 in addition to the closed circuit, the liquid leakage in wide region can be achieved and thus it is possible to improve the reliability of the blood purification apparatus.

According to this embodiment (also same in following embodiment), it is possible to detect liquid leakage at connections between the blood circuit 1 and other structural elements or liquid flow paths forming the blood purification apparatus, or at connections between the dialysate introducing line L1 or the dialysate discharging line L2 and other structural elements or liquid flow paths forming the blood purification apparatus. For example, it is possible to detect liquid leakages at connections between the dialysate introducing port 4c and dialysate introducing line L1 and between the dialysate discharging port 4d and the dialysate discharging line L2 in addition to connections between the blood introducing port 4a of the dialyzer 4 and arterial blood circuit 1a and between blood discharging port 4b and the venous blood circuit 1b.

In the first embodiment, although it is described that the pressure varying step is performed under the condition in which the priming solution is filled in the blood circuit 1, it may be performed under a condition in which air before the priming is filled in the blood circuit 1. In this case, the liquid leakage is detected so that the pressure varying means varies atmosphere (pressure) in the closed circuit and the liquid leakage detecting means detects the liquid leakage in accordance with the atmosphere variation.

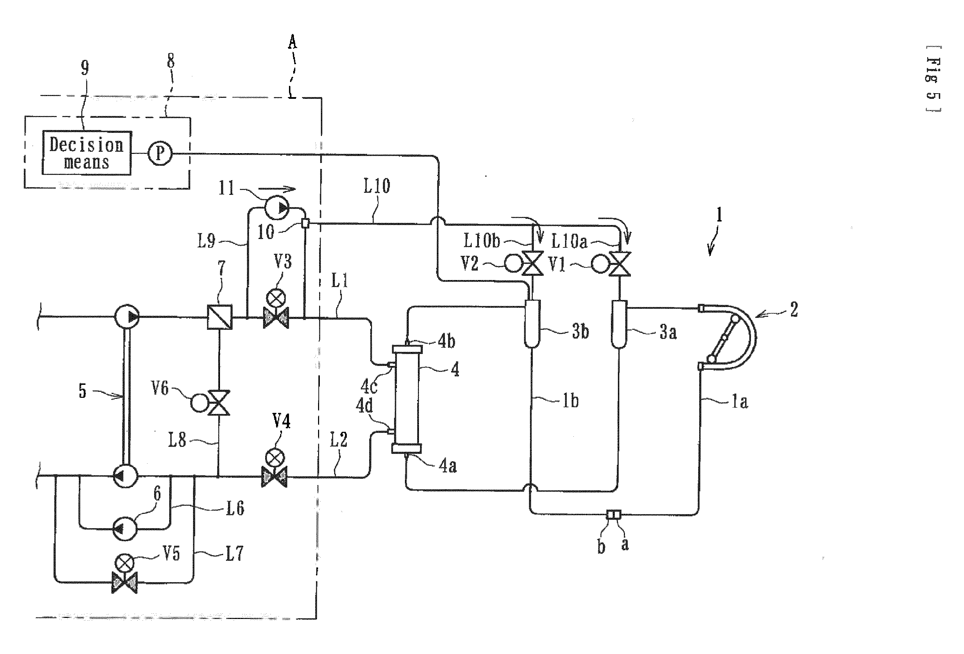

Then a blood purification apparatus of a second embodiment of the present invention will be described.
Similarly to the first embodiment, the blood purification apparatus of this embodiment is adapted to be applied to a hemodialysis apparatus and mainly comprises, as shown in Figs. 5∼7, a blood circuit 1 including an arterial blood circuit 1a and a venous blood circuit 1b, a dialyzer 4 functioning as a blood purification instrument, a dialysate introducing line L1 and a dialysate discharging line L2, a duplex pump 5 and a ultrafiltration pump 6 functioning as liquid supplying means forming a pressure varying means, and a liquid leakage detecting means 8. Same reference numerals will be also used herein for designating same structural elements in the first embodiment and detailed description of them will be omitted.

A bypass line L9 bypassing the electromagnetic valve 3 is connected to the dialysate introducing line L1, and a substitution infusing pump 11 and a substitution infusing port 10 are arranged on the way of the bypass line L9, and the base end of a substitution infusing line L10 is connected to the substitution infusing port 10. The tip end of the substitution infusing line L10 is connected to the top portions (air layer) of the arterial air trap chamber 3a and the venous air trap chamber 3b via a branch line L10a and a branch line L10b respectively. The substitution infusing pump 11 is formed of a displacement type pump and adapted to introduce dialysate to the blood circuit 1 during the blood purification treatment.

When driving the substitution infusing pump 11 during the blood purification treatment, the dialysate flowing through the dialysate introducing line L1 is introduced to the blood circuit 1 via the bypass line L9, the substitution infusing line L10 and the branch lines L10a, L10b and thus the substitution infusion is performed. For performing the pre-dialysate during the blood purification treatment, the dialysate functioning as substitution is introduced to the arterial blood circuit 1a with opening the electromagnetic valve V1 and closing the electromagnetic valve V2, and for performing the post-dialysate during the blood purification treatment, the dialysate functioning as substitution is introduced to the venous blood circuit 1b with closing the electromagnetic valve V1 and opening the electromagnetic valve V2.

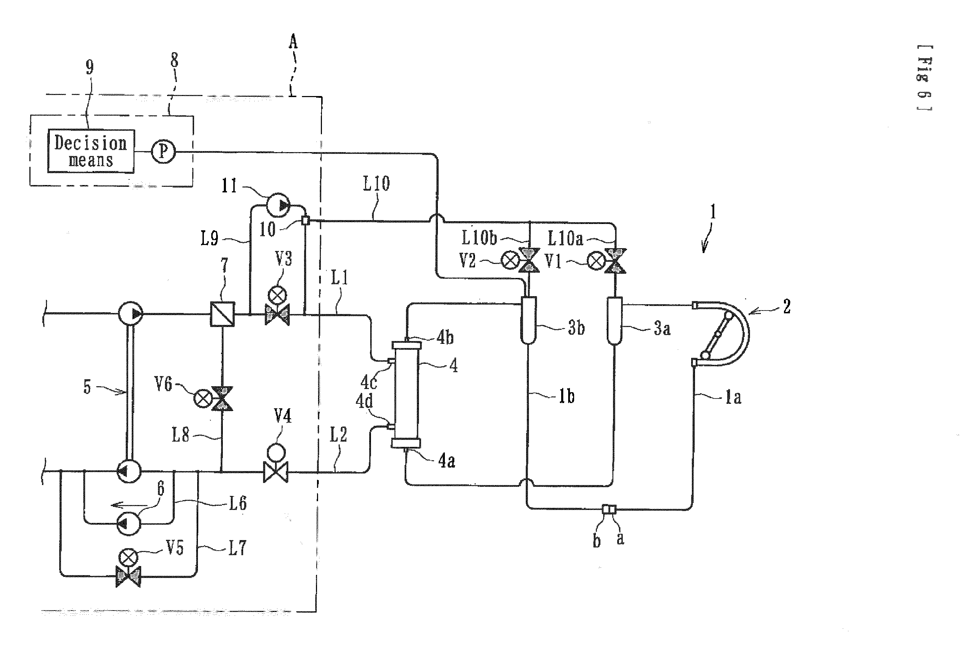

As shown in Figs. 5 and 6, the blood purification apparatus of this embodiment comprises a pressure varying means for varying pressure in a closed circuit under a condition in which the blood circuit 1 is formed as a closed circuit of a sealed condition with connecting the tip end (connector) "a" of the arterial blood circuit 1a and the tip end (connector) "b" of the venous blood circuit 1b and under a condition in which the blood circuit 1 is filled with priming solution, and a liquid leakage detecting means 8 for detecting liquid leakage in the blood circuit 1 in accordance with pressure variation generated by the pressure varying means.

The pressure varying means of the present invention comprises the substitution infusing pump (liquid supplying means) 11 for increasing liquid pressure in the closed circuit by applying a positive pressure to the closed circuit with introducing liquid (dialysate) into the closed circuit from the outside of the closed circuit, and the ultrafiltration pump (liquid supplying means) 6 for decreasing liquid pressure in the closed circuit by applying a negative pressure to the closed circuit with extracting liquid (priming solution (dialysate)) from the closed circuit to the outside of the closed circuit. That is, according to the present invention, the pressure varying means is constructed so that it is possible to increase the liquid pressure in the closed circuit by applying a positive pressure to the closed circuit with introducing liquid (dialysate) into the closed circuit from the outside thereof using the substitution infusing pump 11, and also possible to decrease the liquid pressure in the closed circuit by applying a negative pressure to the closed circuit with extracting liquid (priming solution) from the closed circuit to the outside thereof using the ultrafiltration pump 6 and thus it is possible to perform the liquid leakage detection using the liquid leakage detecting means 8 in the application of both the positive pressure and the negative pressure.

As described above, it is possible to apply the positive pressure to the closed circuit by driving the substitution infusing pump (liquid supplying means) 11 with closing the electromagnetic valves V3, V4 and V5 and opening other electromagnetic valves as shown in Fig. 5 and thus by introducing the dialysate in the dialysate introducing line L1 and the bypass line L9 to the arterial air trap chamber 3a and the venous air trap chamber 3b through the substitution infusing line L10 and branch lines L10a, L10b respectively.

On the contrary, it is possible to apply the negative pressure to the closed circuit by forming the closed circuit of sealed condition in the blood circuit 1 with closing the electromagnetic valves V1, V2 and accordingly the branch lines L10a, L10b and normally rotating the ultrafiltration pump (liquid supplying means) 6 with closing the electromagnetic valves V3, V5 and V6 and opening other electromagnetic valves as shown in Fig. 6 after the priming having been performed with filling the blood circuit 1 with priming solution (e.g. dialysate or physiological saline) under the connected condition of the tip end (connector "a") of the arterial blood circuit 1a and the tip end (connector "b") of the venous blood circuit 1b, and thus by extracting (i.e. normally filtrating) the priming solution in the closed circuit to the dialysate discharging line L2 through the filtration membrane (hollow fiber membrane in the present embodiment) of the dialyzer 4. In this case, the substitution infusing pump 11 is stopped when applying the negative pressure to the closed circuit by normally driving the ultrafiltration pump (liquid supplying means) 6. As described above, since the substitution infusing pump 11 is a displacement type pump, a flow path on which the substitution infusing pump 11 is arranged is closed to prevent any flow of the dialysate.

Similarly to the first embodiment, the liquid leakage detecting means 8 comprises the venous pressure sensor P as liquid pressure detecting means and a decision means 9. As previously described, the venous pressure sensor (liquid pressure detecting means) P is able to continuously (in real time) detect the venous pressure during the blood purification treatment (hemodialysis treatment) and additionally able to continuously (in real time) detect the liquid pressure after the positive pressure having been applied to the closed circuit by the substitution infusing pump 11 and after the negative pressure having been applied to the closed circuit by the ultrafiltration pump 6.

The decision means 9 is formed of e.g. a microcomputer etc. arranged within the main body "A" of the hemodialysis apparatus and electrically connected to the venous pressure sensor (liquid pressure detecting means) P to decide existence of liquid leakage in the blood circuit 1 on the basis of liquid pressure detected by the venous pressure detecting means P. That is, the existence of liquid leakage is decided by the decision means 9 in accordance with tendency after variation of the detected liquid pressure with continuously (in real time) detecting the tendency after variation of the liquid pressure caused by the pressure varying means using the venous pressure sensor P. The method for deciding the liquid leakage is same as that of the first embodiment.

Then, the method for inspecting liquid leakage of a dialysis apparatus of the second embodiment will be described.
Firstly, the liquid pressure in the closed circuit is varied by the pressure varying means under a condition in which the blood circuit 1 is formed as a closed circuit of a sealed condition with connecting the tip end of the arterial blood circuit 1a and the tip end of the venous blood circuit 1b and under a condition in which the blood circuit 1 is filled with priming solution (pressure varying step). Then, the liquid leakage in the blood circuit 1 is detected by the liquid leakage detecting means in accordance with the variation of the liquid pressure in the pressure varying step (liquid leakage detecting step).

The liquid leakage detecting step comprises a pressure detecting step for detecting liquid pressure in the closed circuit after a positive pressure or a negative pressure has been applied to the closed circuit; and a decision step for deciding existence of liquid leakage in the blood circuit 1 on the basis of the liquid pressure detected in the pressure detecting step. In addition, in the pressure varying step, the liquid pressure in the closed circuit is increased by applying a positive pressure to the closed circuit by driving the substitution infusing pump 11 with introducing liquid into the closed circuit from the outside thereof and also decreased by applying a negative pressure to the closed circuit by normally rotating the ultrafiltration pump 6 with extracting liquid from the closed circuit to the outside thereof. When the liquid leakage is detected in the liquid leakage detecting step, the fact is informed to an operator to urge him confirming the liquid leakage.

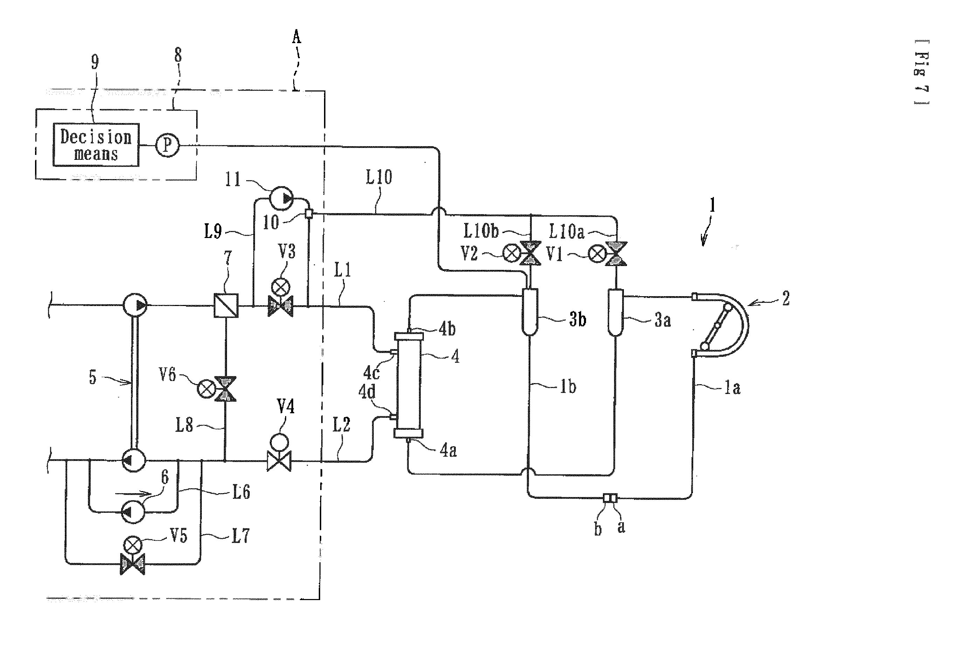

It is preferable that the ultrafiltration pump 6 functioning as the liquid supplying means is a pump being able to rotate both in normal and reverse directions so that either a positive pressure or a negative pressure can be applied to the closed circuit by selectively driving the ultrafiltration pump 6 in the normal direction or reverse direction. That is, as previously described, when the ultrafiltration pump 6 is normally rotated, it is possible to apply the negative pressure to the closed circuit. On the contrary, as shown in Fig. 7, when the ultrafiltration pump 6 is reversely rotated, the dialysate in the dialysate discharging line L2 is introduced to the flow path of blood from the flow path of dialysate of the dialyzer 4 through the filtration membrane (hollow fiber membrane in the present embodiment) (inverse filtration). Thus, the positive pressure can be applied to the closed circuit. According to such a construction, it is possible to easily and arbitrary apply the positive pressure and negative pressure to the closed circuit.

As described above, the pressure varying means and the pressure varying step can vary the liquid pressure in the closed circuit by introducing liquid to the closed circuit from the substitution infusing line L10 or by extracting liquid from the closed circuit to the dialysate discharging line L2, and the liquid leakage detecting means and the liquid leakage detecting step can detect the liquid leakage of the substitution infusing line L10 or the dialysate discharging line L2 (strictly, liquid leakage in a positive pressure applying region of the substitution infusing line L10 or in a negative pressure applying region of the dialysate discharging line L2) in addition to the closed circuit. Accordingly, since it is possible to detect liquid leakage of the substitution infusing line L10 or the dialysate discharging line L2 in addition to the closed circuit, the liquid leakage in wide region can be achieved and thus it is possible to improve the reliability of the blood purification apparatus.

In the second embodiment, although it is described that the pressure varying step is performed under the condition in which the priming solution is filled in the blood circuit 1, it may be performed under a condition in which air before the priming is filled in the blood circuit 1. In this case, the liquid leakage is detected so that the pressure varying means varies atmosphere (pressure) in the closed circuit and the liquid leakage detecting means detects the liquid leakage in accordance with the atmosphere variation.

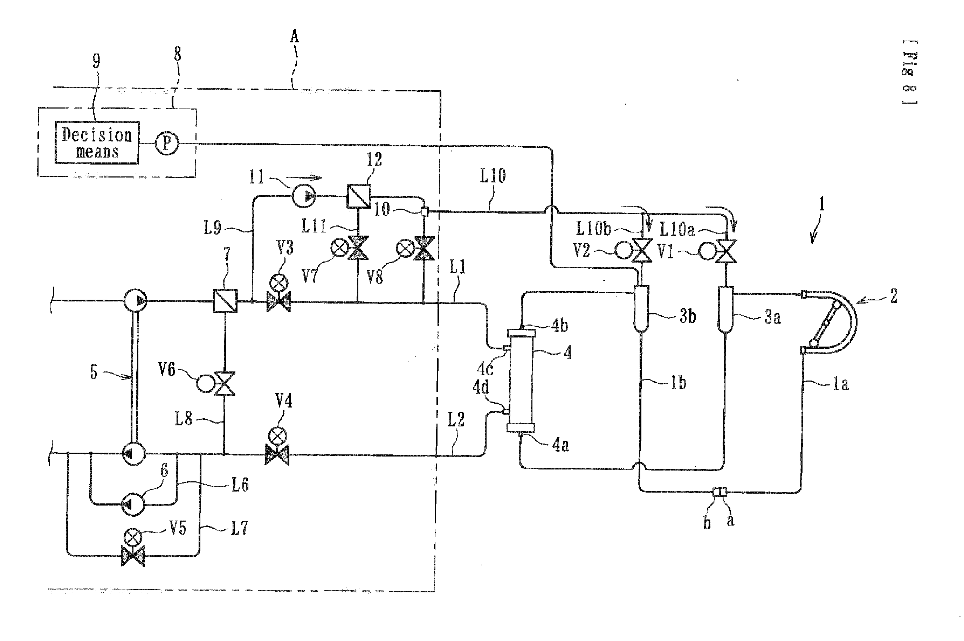

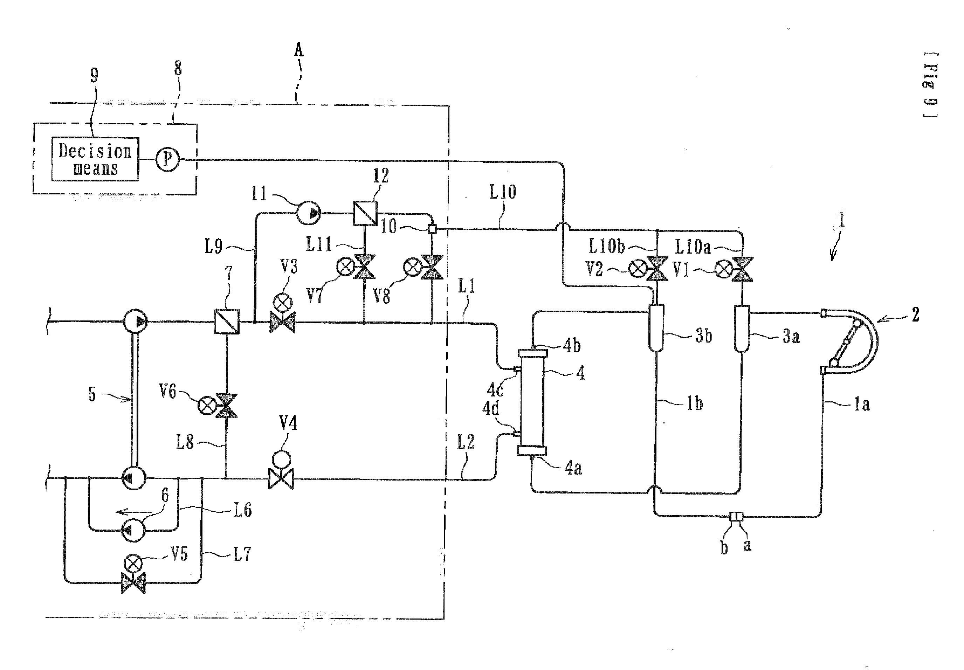

Then a blood purification apparatus of a third embodiment of the present invention will be described.
Similarly to the first and second embodiments, the blood purification apparatus of this embodiment is adapted to be applied to a hemodialysis apparatus and mainly comprises, as shown in Figs. 8 and 9, a blood circuit 1 including an arterial blood circuit 1a and a venous blood circuit 1b, a dialyzer 4 functioning as a blood purification instrument, a dialysate introducing line L1 and a dialysate discharging line L2, a duplex pump 5 and a ultrafiltration pump 6 functioning as liquid supplying means forming a pressure varying means, and a liquid leakage detecting means 8. Same reference numerals will be also used herein for designating same structural elements in the first and second embodiments and detailed description of them will be omitted.

Similarly to the second embodiment, a bypass line L9 bypassing the electromagnetic valve 3 is connected to the dialysate introducing line L1, and a substitution infusing pump 11 and a substitution infusing port 10 are arranged on the way of the bypass line L9, and the base end of a substitution infusing line L10 is connected to the substitution infusing port 10. The tip end of the substitution infusing line L10 is connected to the top portions (air layer) of the arterial air trap chamber 3a and the venous air trap chamber 3b via a branch line L10a and a branch line L10b respectively.

An electromagnetic valve V8 is arranged between the substitution infusing port 10 and the dialysate introducing line L1 on the bypass line L9. In addition, a flow path L11 is arranged between a filter 12 arranged on the bypass line L9 and the dialysate introducing line L1 and an electromagnetic valve V7 is arranged on the flow path L11.

When driving the substitution infusing pump 11 during the blood purification treatment, the dialysate flowing through the dialysate introducing line L1 is introduced to the blood circuit 1 via the substitution infusing line L10 and the branch lines L10a, L10b after having been filtrated by the filter 12 during flowing through the bypass line L9 and thus the substitution infusion is performed. Similarly to the second embodiment, for performing the pre-dialysate during the blood purification treatment, the dialysate functioning as substitution is introduced to the arterial blood circuit 1a with opening the electromagnetic valve V1 and closing the electromagnetic valve V2, and for performing the post-dialysate during the blood purification treatment, the dialysate functioning as substitution is introduced to the venous blood circuit 1b with closing the electromagnetic valve V1 and opening the electromagnetic valve V2.

As shown in Figs. 8 and 9, the blood purification apparatus of this embodiment comprises a pressure varying means for varying pressure in a closed circuit under a condition in which the blood circuit 1 is formed as a closed circuit of a sealed condition with connecting the tip end (connector) "a" of the arterial blood circuit 1a and the tip end (connector) "b" of the venous blood circuit 1b and under a condition in which the blood circuit 1 is filled with priming solution, and a liquid leakage detecting means 8 for detecting liquid leakage in the blood circuit 1 in accordance with pressure variation generated by the pressure varying means.

The pressure varying means of the present invention comprises the substitution infusing pump (liquid supplying means) 11 for increasing liquid pressure in the closed circuit by applying a positive pressure to the closed circuit with introducing liquid (dialysate) into the closed circuit from the outside of the closed circuit, and the ultrafiltration pump (liquid supplying means) 6 for decreasing liquid pressure in the closed circuit by applying a negative pressure to the closed circuit with extracting liquid (priming solution (dialysate)) from the closed circuit to the outside of the closed circuit. That is, according to the present invention, the pressure varying means is constructed so that it is possible to increase the liquid pressure in the closed circuit by applying a positive pressure to the closed circuit with introducing liquid (dialysate) into the closed circuit from the outside thereof using the substitution infusing pump 11, and also possible to decrease the liquid pressure in the closed circuit by applying a negative pressure to the closed circuit with extracting liquid (priming solution) from the closed circuit to the outside thereof using the ultrafiltration pump 6 and thus it is possible to perform the liquid leakage detection using the liquid leakage detecting means 8 in the application of both the positive pressure and the negative pressure.

As described above, it is possible to apply the positive pressure to the closed circuit by driving the substitution infusing pump (liquid supplying means) 11 with closing the electromagnetic valves V3, V4, V5, V7 and V8 and opening other electromagnetic valves as shown in Fig. 8 and thus by introducing the dialysate in the dialysate introducing line L1 and the bypass line L9 to the arterial air trap chamber 3a and the venous air trap chamber 3b through the substitution infusing line L10 and branch lines L10a, L10b respectively.

On the contrary, it is possible to apply the negative pressure to the closed circuit by forming the closed circuit of sealed condition in the blood circuit 1 with closing the electromagnetic valves V1, V2 and accordingly the branch lines L10a, L10b and normally rotating the ultrafiltration pump (liquid supplying means) 6 with closing the electromagnetic valves V3, V5, V6, V7 and V8 and opening other electromagnetic valves as shown in Fig. 9 after the priming having been performed with filling the blood circuit 1 with priming solution (e.g. dialysate or physiological saline) under the connected condition of the tip end (connector "a") of the arterial blood circuit 1a and the tip end (connector "b") of the venous blood circuit 1b, and thus by extracting (i.e. normally filtrating) the priming solution in the closed circuit to the dialysate discharging line L2 through the filtration membrane (hollow fiber membrane in the present embodiment) of the dialyzer 4. In this case, the substitution infusing pump 11 is stopped when applying the negative pressure to the closed circuit by normally driving the ultrafiltration pump (liquid supplying means) 6.

Similarly to the second embodiment, the liquid leakage detecting means 8 comprises the venous pressure sensor P as a liquid pressure detecting means and a decision means 9. As previously described, the venous pressure sensor (liquid pressure detecting means) P is able to continuously (in real time) detect the venous pressure during the blood purification treatment (hemodialysis treatment) and additionally able to continuously (in real time) detect the liquid pressure after the positive pressure having been applied to the closed circuit by the substitution infusing pump 11 and after the negative pressure having been applied to the closed circuit by the ultrafiltration pump 6.

The decision means 9 is formed of e.g. a microcomputer etc. arranged within the main body "A" of the hemodialysis apparatus and electrically connected to the venous pressure sensor (liquid pressure detecting means) P to decide existence of liquid leakage in the blood circuit 1 on the basis of liquid pressure detected by the venous pressure detecting means P. That is, the existence of liquid leakage is decided by the decision means 9 in accordance with tendency after variation of the detected liquid pressure with continuously (in real time) detecting the tendency after variation of the liquid pressure caused by the pressure varying means using the venous pressure sensor P. The method for deciding the liquid leakage is same as that of the first embodiment.

Then, the method for inspecting liquid leakage of a dialysis apparatus of the third embodiment will be described.
Firstly, the liquid pressure in the closed circuit is varied by the pressure varying means under a condition in which the blood circuit 1 is formed as a closed circuit of a sealed condition with connecting the tip end of the arterial blood circuit 1a and the tip end of the venous blood circuit 1b and under a condition in which the blood circuit 1 is filled with priming solution (pressure varying step). Then, the liquid leakage in the blood circuit 1 is detected by the liquid leakage detecting means in accordance with the variation of the liquid pressure in the pressure varying step (liquid leakage detecting step).

The liquid leakage detecting step comprises a pressure detecting step for detecting liquid pressure in the closed circuit after a positive pressure or a negative pressure has been applied to the closed circuit; and a decision step for deciding existence of liquid leakage in the blood circuit 1 on the basis of the liquid pressure detected in the pressure detecting step. In addition, in the pressure varying step, the liquid pressure in the closed circuit is increased by applying a positive pressure to the closed circuit by driving the substitution infusing pump 11 with introducing liquid into the closed circuit from the outside thereof and also decreased by applying a negative pressure to the closed circuit by normally rotating the ultrafiltration pump 6 with extracting liquid from the closed circuit to the outside thereof. Similarly to the first and second embodiments, it is preferable that the ultrafiltration pump 6 functioning as the liquid supplying means is a pump being able to rotate both in normal and reverse directions so that either a positive pressure or a negative pressure can be applied to the closed circuit by selectively driving the ultrafiltration pump 6 in the normal direction or reverse direction. When the liquid leakage is detected in the liquid leakage detecting step, the fact is informed to an operator to urge him confirming the liquid leakage.

As described above, the pressure varying means and the pressure varying step can vary the liquid pressure in the closed circuit by introducing liquid to the closed circuit from the substitution infusing line L10 or by extracting liquid from the closed circuit to the dialysate discharging line L2, and the liquid leakage detecting means and the liquid leakage detecting step can detect the liquid leakage of the substitution infusing line L10 or the dialysate discharging line L2 (strictly, liquid leakage in a positive pressure applying region of the substitution infusing line L10 or in a negative pressure applying region of the dialysate discharging line L2) in addition to the closed circuit. Accordingly, since it is possible to detect liquid leakage of the substitution infusing line L10 or the dialysate discharging line L2 in addition to the closed circuit, the liquid leakage in wide region can be achieved and thus it is possible to improve the reliability of the blood purification apparatus.

In the third embodiment, although it is described that the pressure varying step is performed under the condition in which the priming solution is filled in the blood circuit 1, it may be performed under a condition in which air before the priming is filled in the blood circuit 1. In this case, the liquid leakage is detected so that the pressure varying means varies atmosphere (pressure) in the closed circuit and the liquid leakage detecting means detects the liquid leakage in accordance with the atmosphere variation.

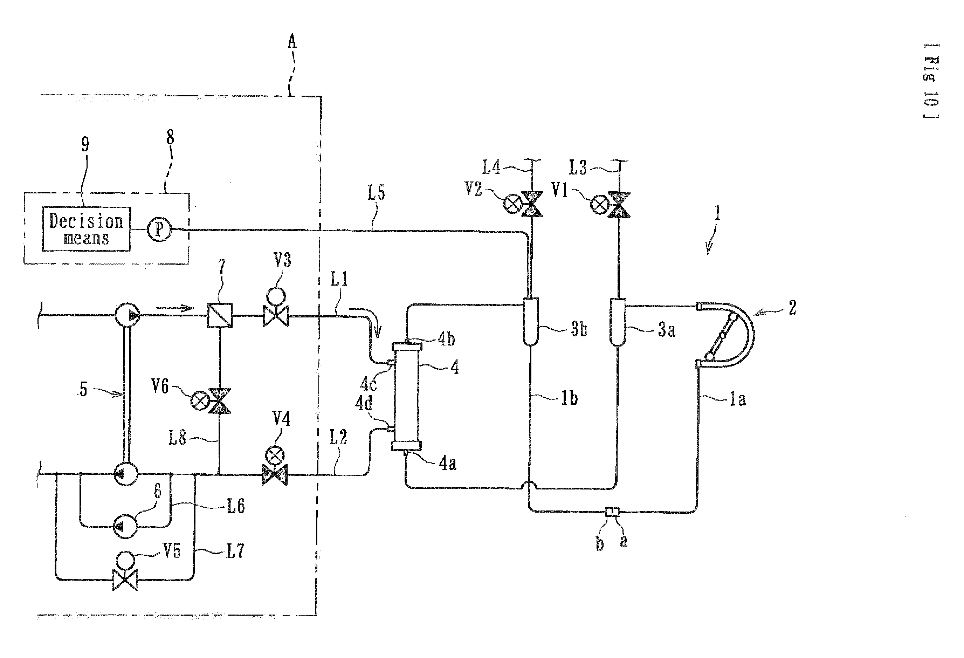

Then a blood purification apparatus of a fourth embodiment of the present invention will be described.
Similarly to the first∼third embodiments, the blood purification apparatus of this embodiment is adapted to be applied to a hemodialysis apparatus and mainly comprises, as shown in Figs. 10∼13, a blood circuit 1 including an arterial blood circuit 1a and a venous blood circuit 1b, a dialyzer 4 functioning as a blood purification instrument, a dialysate introducing line L1 and a dialysate discharging line L2, a duplex pump 5 functioning as liquid supplying means forming a pressure varying means, a liquid leakage detecting means 8, and overflow lines L3, L4 as a discharging line. Same reference numerals will be also used herein for designating same structural elements in the first embodiment and detailed description of them will be omitted.

The blood purification apparatus of this embodiment comprises a pressure varying means for varying pressure in a closed circuit under a condition in which the blood circuit 1 is formed as a closed circuit of a sealed condition with connecting the tip end (connector) "a" of the arterial blood circuit 1a and the tip end (connector) "b" of the venous blood circuit 1b, and a liquid leakage detecting means 8 arranged in the closed circuit for detecting liquid leakage in the blood circuit 1 in accordance with pressure variation generated by the pressure varying means. The pressure varying means of the present invention comprises the duplex pump (liquid supplying means) 5 for increasing liquid pressure in the closed circuit by applying a positive pressure to the closed circuit with introducing liquid (dialysate) into the closed circuit from the outside of the closed circuit.

As described above, under the condition in which the tip end (connector) "a" of the arterial blood circuit 1a and the tip end (connector) "b" of the venous blood circuit 1b, a closed and sealed circuit is formed in the blood circuit 1 (in this embodiment a closed circuit filled with air before priming) with closing the electromagnetic valves V1, V2 to close the overflow lines L3, L4 as shown in Fig. 10. In such a condition, when driving the duplex pump (liquid supplying means) 5, dialysate in the dialysate introducing line L1 (portion between the duplex pump 5 and the dialyzer 4) and the dialysate discharging line L2 (portion between the dialyzer 4 and the electromagnetic valve V4) can be introduced to a blood flow path from a dialysate flow path of the dialyzer 4 through the filtration membrane (hollow fiber membrane in the present embodiment) of the dialyzer 4 (inverse nitration). Thus, it is possible to apply the positive pressure to the closed circuit.

The overflow lines L3, L4 as discharging lines comprise flow paths being able to discharge liquid or gas outside of the closed circuit and able to be opened and closed by the electromagnetic valves V1, V2. That is, the overflow lines L3, L4 are closed by closing the electromagnetic valves V1, V2 and thus the closed circuit is formed. On the other hand, when opening either or one of the electromagnetic valves V1, V2, the closed condition of the overflow lines L3, L4 are released and thus liquid or gas in the is discharged outside.

Then, the method for inspecting liquid leakage of a dialysis apparatus of the fourth embodiment will be described.
Firstly the liquid pressure in the closed circuit is varied by the pressure varying means under a condition in which the blood circuit 1 is formed as a closed circuit of a sealed condition (pre-priming condition) with connecting the tip end of the arterial blood circuit 1a and the tip end of the venous blood circuit 1b (pressure varying step). Then, the liquid leakage in the blood circuit 1 is detected by the liquid leakage detecting means in accordance with the variation of the liquid pressure in the pressure varying step (liquid leakage detecting step).

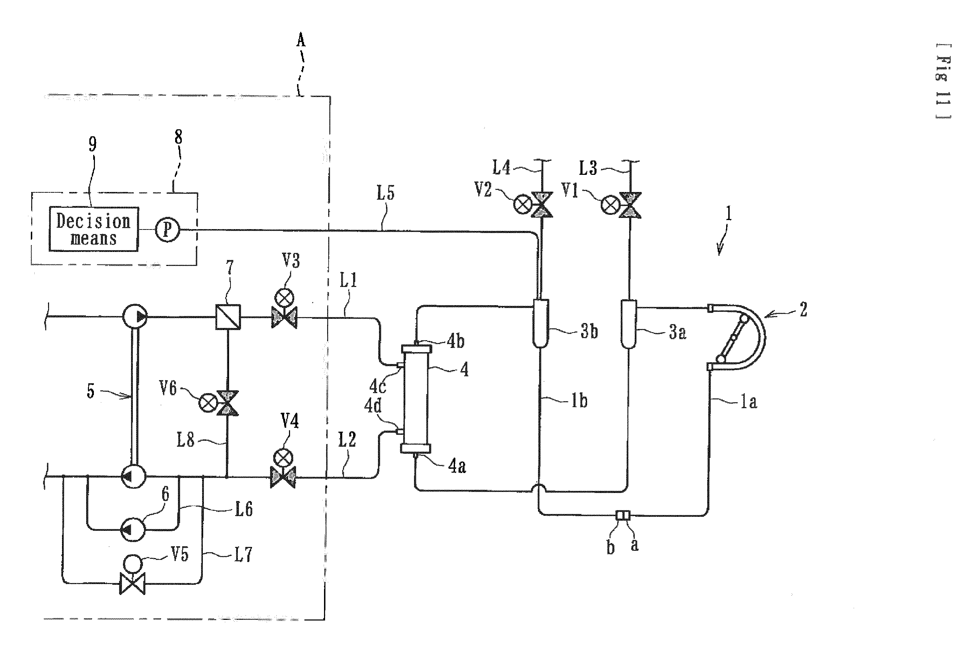

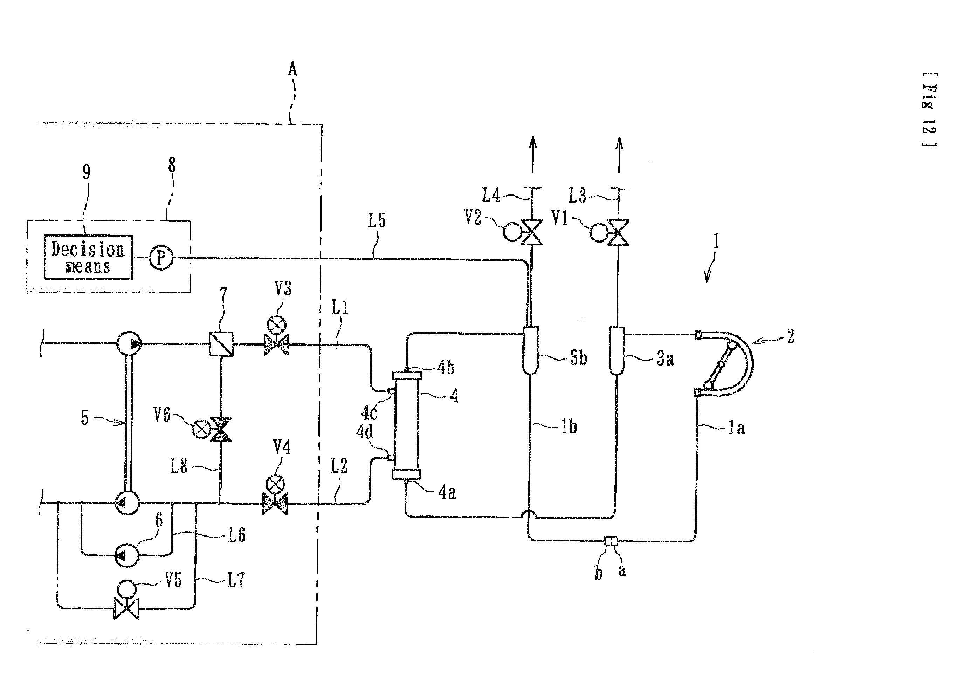

The pressure varying step of this embodiment is constructed so that the liquid leakage detecting step (see Fig. 11) for detecting the liquid leakage in the blood circuit by the liquid leakage detecting means during the pressurizing step is performed with alternately performing the pressurizing step (see Fig. 10) in which the positive pressure is applied to the closed circuit by introducing liquid (dialysate) into the closed circuit from the outside thereof by the duplex pump (liquid supplying means) 5 with closing the overflow lines L3, L4, and the priming step (see Fig. 12) in which liquid or gas in the closed circuit is discharged from the overflow lines L3, L4 with opening the overflow lines L3, L4 and introducing liquid to the closed circuit from the outside thereof by the duplex pump 5.

More particularly, as shown in Fig. 10, the pressurizing step is a step intended to increase the pressure in the closed circuit by applying the positive pressure to the closed circuit with closing the V1, V2, V4 and V6 and opening other electromagnetic valves and with inverse filtrating dialysate in the dialysate introducing line L1 by the duplex pump 5. In the pressurizing step, it is possible to apply the positive pressure to the closed circuit by introducing dialysate to the closed circuit with reversely rotating the ultrafiltration pump 6 in place of the duplex pump 5 (in which the electromagnetic valve V4 being opened and the electromagnetic valve V3 being closed).

The decision step is a step performed during the pressurizing step and constructed as shown in Fig. 11 so that the existence of liquid leakage in the blood circuit 1 is decided by detecting the pressure in the closed circuit by the venous pressure sensor P (pressure detecting means) with closing the electromagnetic valve V3 and by the decision means 9 based on the detected value. In the decision step, although the duplex pump 5 is kept a stopped condition, it is possible to keep the operation of the duplex pump 5 with opening the electromagnetic valve V6.

According to this embodiment, since a portion between the dialyzer 4 and the electromagnetic valve V3 on the dialysate introducing line L1 and a portion between the dialyzer 4 and the electromagnetic valve V4 on the dialysate discharging line L2 are included in the closed circuit in addition to the blood circuit 1, it is possible to decide existence of liquid leakage of the dialysate introducing line L1 and the dialysate discharging line L2.

The priming step is a step performed after the pressurizing step and constructed as shown in Fig. 12 so that the pressure in the closed circuit is released to e.g. the normal pressure by discharging outside gas (air) or liquid (dialysate) in the closed circuit from the overflow lines L3, L4 with opening the electromagnetic valves V1, V2. That is, it is possible to discharge air (including air bubbles in the dialysate) in the closed circuit from the overflow lines L3, L4 by the priming step. Similarly to the liquid leakage detecting step, also in the priming step, although the duplex pump 5 is kept a stopped condition, it is possible to keep the operation of the duplex pump 5 with opening the electromagnetic valve V6.

According to this embodiment, the dialysate functioning as priming solution is filled in the closed circuit with alternately repeating a predetermined times the pressurizing step and the priming step. However, since it is difficult to fill with dialysate a portion between the arterial air trap chamber 3a and the venous air trap chamber 3b, a circulating step is performed as follows.

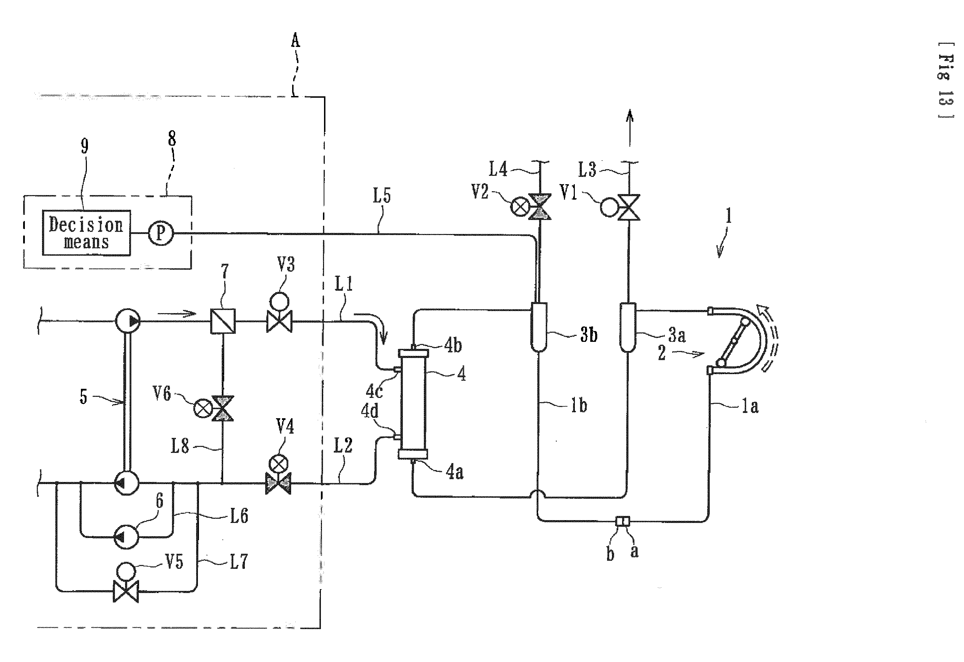

As shown in Fig. 13, the circulating step is a step for discharging air (including air bubbles in the dialysate) in the closed circuit from the overflow line L3 by driving the duplex pump 5 and the blood pump 2 to circulate dialysate in the closed circuit with opening the electromagnetic valve V1 and closing the electromagnetic valve V2 as well as with closing the electromagnetic valves V4, V6 and opening other electromagnetic valves. Such a circulating step enables to fill all flowing paths forming the closed circuit with dialysate as the priming solution.

As described above, in this embodiment, since the dialysate as priming solution is filled in the closed circuit by alternately repeating predetermined times the pressurizing step and the priming step, the pressurizing step is usually performed several times, the liquid leakage detecting step may be performed at all times of performing the pressurizing step or may be performed at only a predetermined time (e.g. at a time of final pressurizing time).

In this case, it is possible to achieve finer liquid leakage detection if the liquid leakage detecting step is performed at all times of a plurality of pressurizing steps, and also possible to achieve more exact liquid leakage detection if the liquid leakage detection step is performed in only case of the priming solution (dialysate) being filled in the closed circuit. In addition, although it is described that the liquid leakage detecting step of this embodiment is performed with keeping the electromagnetic valve V3 close (see Fig. 11), it may be possible to perform the liquid leakage detection with keeping the opened condition of the electromagnetic valve V3 in the pressurizing step.

According to the first∼fourth embodiments of the present invention, since the liquid leakage in the blood circuit 1 is detected by varying pressure in the closed circuit formed by connecting the tip end of the arterial blood circuit and the tip end of the venous blood circuit, it is possible to perform a sufficient liquid leakage inspection over a whole region(whole region including the tip end side of the arterial blood circuit 1a and the tip end side of the venous blood circuit 1b) of the flow path of the blood circuit 1. More particularly according to the first∼ third embodiments, since the liquid leakage in the blood circuit 1 is detected by varying the liquid pressure in the closed circuit under a condition in which the blood circuit 1 is filled with priming liquid, it is possible to perform a sufficient liquid leakage inspection over a whole region of the blood circuit 1.

According to the fourth embodiment, since the blood purification apparatus further comprises overflow lines L3, L4 as discharging lines for discharging liquid or gas in the closed circuit to the outside thereof; and the liquid leakage in the blood circuit 1 is detected during a pressurizing step by alternately performing the pressurizing step and a priming step, in the pressurizing step the positive pressure being applied to the closed circuit by introducing liquid into the closed circuit from the outside thereof by the duplex pump 5 as a liquid supplying means with keeping a closed condition of the overflow lines L3, L4, and in the priming step liquid being introduced into the closed circuit from the outside thereof by the duplex pump 5 with keeping a opened condition of the overflow lines L3, L4 and then liquid or gas in the closed circuit being discharged from the overflow lines L3, L4, it is possible to perform the detection of liquid leakage during a process in which the priming (discharge of air bubbles and filling of priming liquid) in the blood circuit 1 is performed.

Furthermore, since the liquid leakage detection is performed by detecting pressure in the closed circuit after a positive pressure has been applied to the closed circuit, and by deciding existence of liquid leakage in the blood circuit 1 on the basis of the detected pressure, it is possible to detect the pressure when the positive pressure is applied to the closed circuit for example by substituting a venous pressure sensor P usually connected to the venous air trap chamber 3b connected to the venous blood circuit 1b for a positive pressure detecting sensor and thus to reduce the manufacturing cost of the blood purification apparatus.

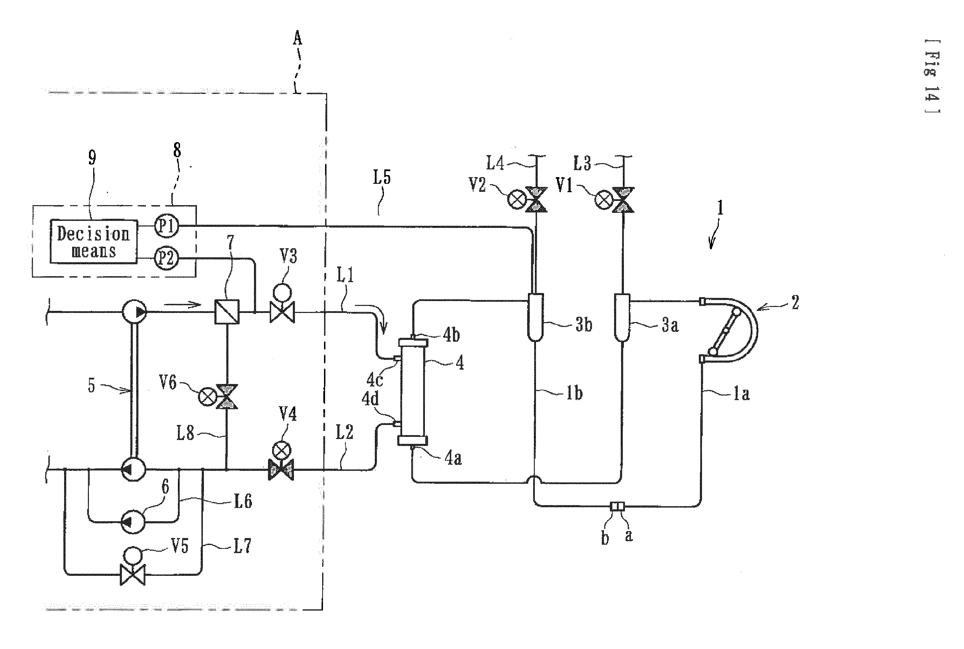

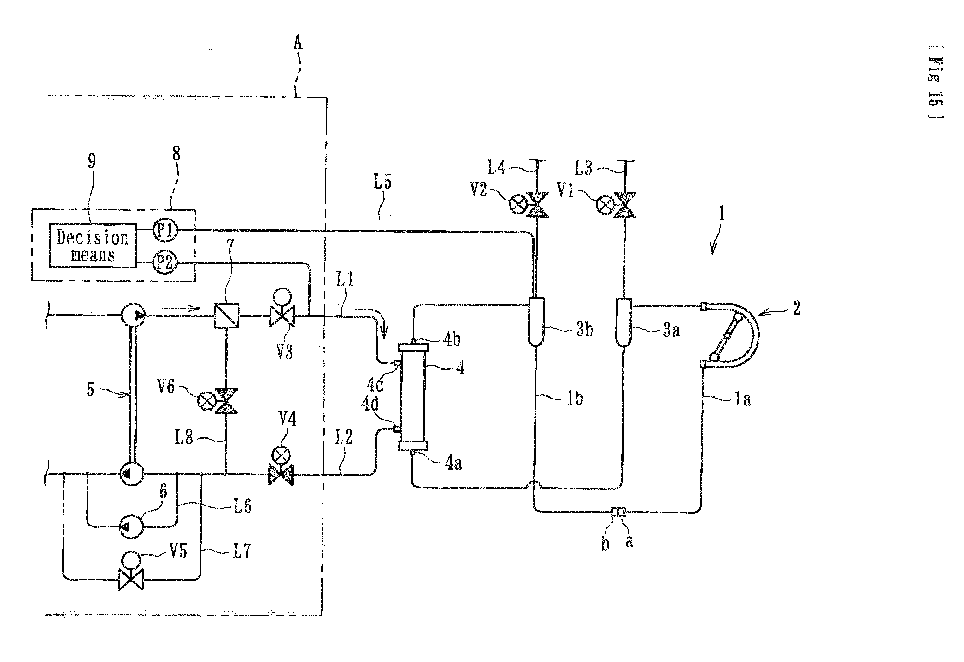

However, the existence of liquid leakage can be detected by modifications shown in Figs. 14 and 15. In the modification of Fig. 14, a pressure detecting means (venous pressure sensor) P1 is connected to the venous air trap chamber 3b in the closed circuit and another pressure detecting means P2 is connected to a portion upstream of the electromagnetic valve V3 (between the electromagnetic valve V3 and the filter 7) on the dialysate introducing line L1, and the existence of liquid leakage can be decided by the decision means 9 with comparing pressures detected by the pressure detecting means P1, P2. In the modification of Fig. 15, a pressure detecting means (venous pressure sensor) P1 is connected to the venous air trap chamber 3b in the closed circuit and another pressure detecting means P2 is connected to a portion downstream of the electromagnetic valve V3 (between the electromagnetic valve V3 and the dialyzer 4) on the dialysate introducing line L1, and the existence of liquid leakage can be decided by the decision means 9 with comparing pressures detected by the pressure detecting means P1, P2.

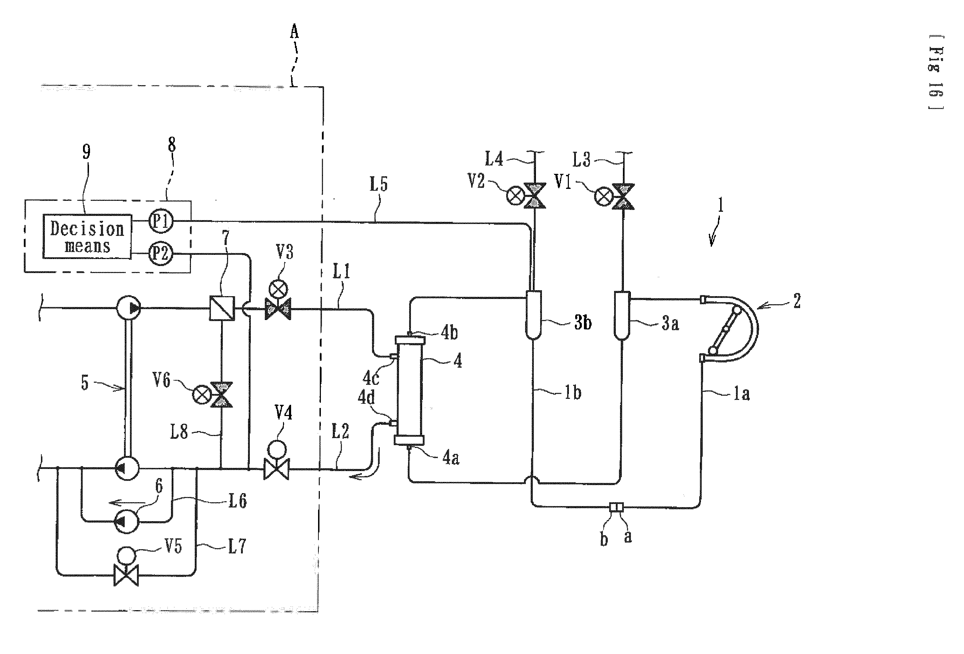

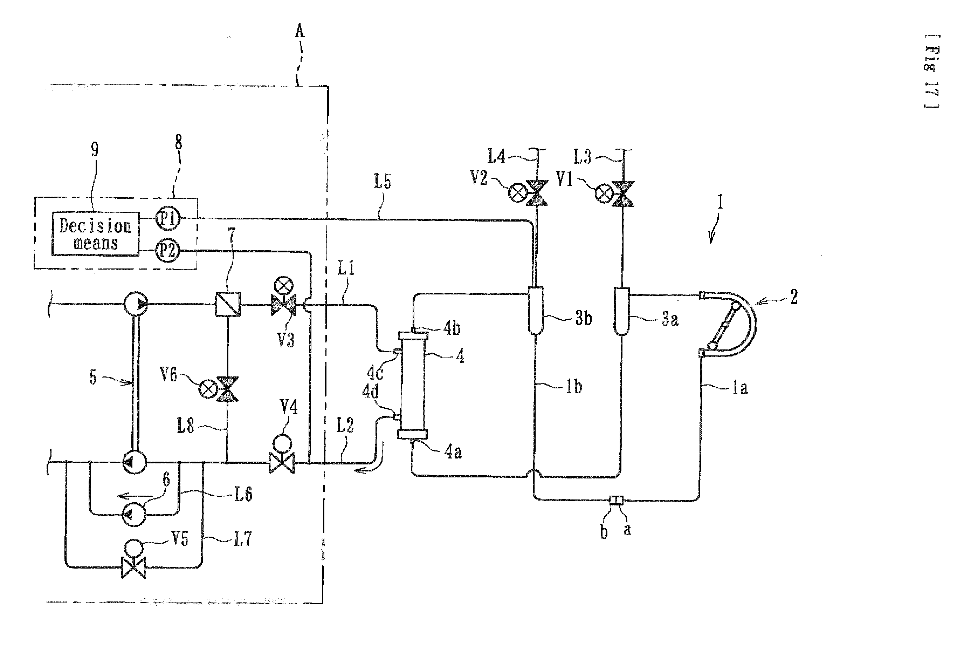

In addition, the existence of liquid leakage can be detected by modifications shown in Figs. 16 and 17. In the modification of Fig. 16, a pressure detecting means (venous pressure sensor) P1 is connected to the venous air trap chamber 3b in the closed circuit and another pressure detecting means P2 is connected to a portion downstream of the electromagnetic valve V4 (between the electromagnetic valve V4 and the bypass line L6) on the dialysate discharging line L2, and the existence of liquid leakage can be decided by the decision means 9 with comparing pressures detected by the pressure detecting means P1, P2. In the modification of Fig. 17, a pressure detecting means (venous pressure sensor) P1 is connected to the venous air trap chamber 3b in the closed circuit and another pressure detecting means P2 is connected to a portion upstream of the electromagnetic valve V4 (between the electromagnetic valve V4 and the dialyzer 4) on the dialysate introducing line L1, and the existence of liquid leakage can be decided by the decision means 9 with comparing pressures detected by the pressure detecting means P1, P2.

As described above, since the pressure detecting means P1, P2 are arranged respectively on the closed circuit and on the dialysate introducing line L1 or the dialysate discharging line L2 and the decision means 9 decides the existence of liquid leakage in the blood circuit 1 with comparing pressures detected by the pressure detecting means P1, P2, it is possible to further improve the accuracy of decision of the liquid leakage.

That is, it is possible to decide malfunction of either one of the pressure detecting means, clog etc. in the flow paths of the blood circuit 1, dialysate introducing line L1 or the dialysate discharging line L2 and thus to improve the deciding accuracy of liquid leakage if monitoring whether a pressure detected by the pressure detecting means P1 corresponds to a pressure detected by the pressure detecting means P2. Thus it is possible to improve the reliability of the blood purification apparatus.

Furthermore, the pressure variation applied to the closed circuit according to the present invention includes both the pressure increase obtained by applying the positive pressure to the closed circuit with introducing liquid (dialysate) to the closed circuit from the outside thereof and the pressure decrease obtained by applying the negative pressure to the closed circuit with extracting liquid (dialysate) from the closed circuit to the outside thereof. Accordingly, since the detection of liquid leakage can be performed when both the positive pressure and the negative pressure are applied, more suitable and sufficient liquid leakage inspection can be achieved. However, in this case it is preferable to apply the positive pressure to the closed circuit after application of the negative pressure since this enables to prevent suction of air from the tip ends of the arterial blood circuit 1a and the venous blood circuit when the connection of them is released and to smoothly perform the blood purification treatment thereafter.

In addition, according to the first embodiment∼fourth embodiment, since the introduction and discharge of liquid to or from the closed circuit is performed by the duplex pump (dialysate pump) 5 for introducing dialysate to the dialyzer (blood purification instrument) 4, the ultrafiltration pump 6 for performing ultrafiltration against blood extracorporeally circulating through the blood circuit 1 or the substitution infusing pump 11, it is possible to substitute pumps used in the blood purification treatment for these pumps and thus to also reduce the manufacturing cost of the blood purification apparatus. The liquid supplying means are not limited to pumps and other means (including those not driven during the blood purification treatment) can be used if they can apply the positive pressure or the negative pressure by introducing or extracting liquid (dialysate) to or from the closed circuit.

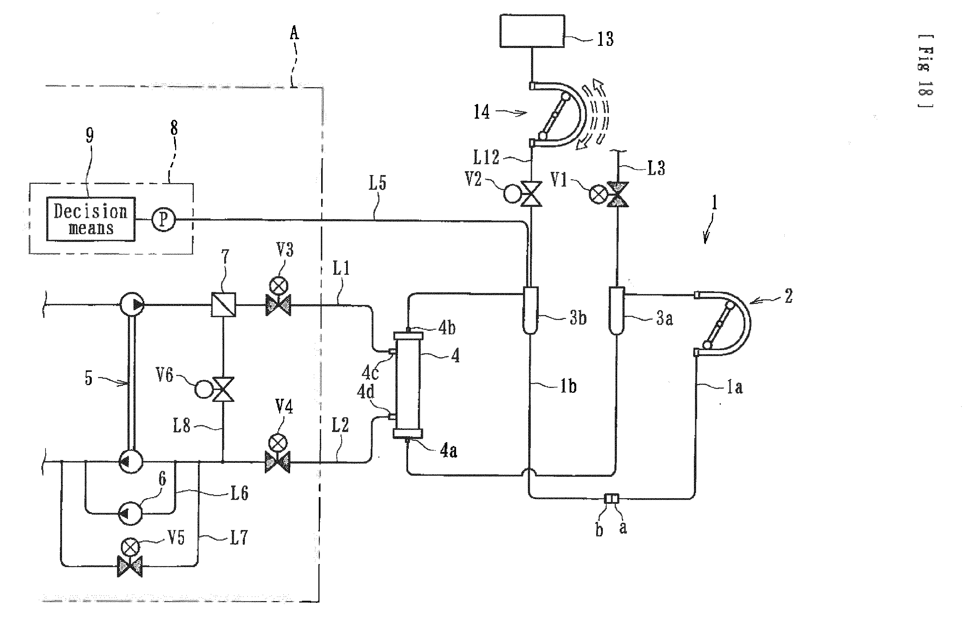

Although preferable embodiments and modifications of the present invention have been described above, the present invention is not limited to these embodiments and modifications. For example as shown in Fig. 18, the present invention can be applied to a construction in which a physiological saline line L12 extends from an upper portion (air layer) of the venous air trap chamber 3b to a physiological saline bag 13 containing a predetermined amount of physiological saline. The electromagnetic valve V2 and a solution infusing pump 14 as a liquid supplying means are arranged on the physiological saline line L12 and thus the physiological saline in the physiological saline bag 13 can be introduced to the venous blood circuit 1b through the venous air trap chamber 3b.

Thus, as shown in Fig.18, it is possible to apply the positive pressure to the closed circuit by closing the electromagnetic valves V1, V3, V4 and V5 and opening other electromagnetic valves and normally rotating the substitution infusing pump (liquid supplying means) 14 and by introducing liquid (physiological saline) in the physiological saline bag 13 to the venous air trap chamber 3b via the physiological saline line L12 after having performed the priming by filling the blood circuit 1 with the priming solution (e.g. physiological saline in the physiological saline bag 13) under a condition of formation of the closed circuit of a sealed condition with connecting the tip end (connector "a") of the arterial blood circuit 1a and the tip end (connector "b") of the venous blood circuit 1b.

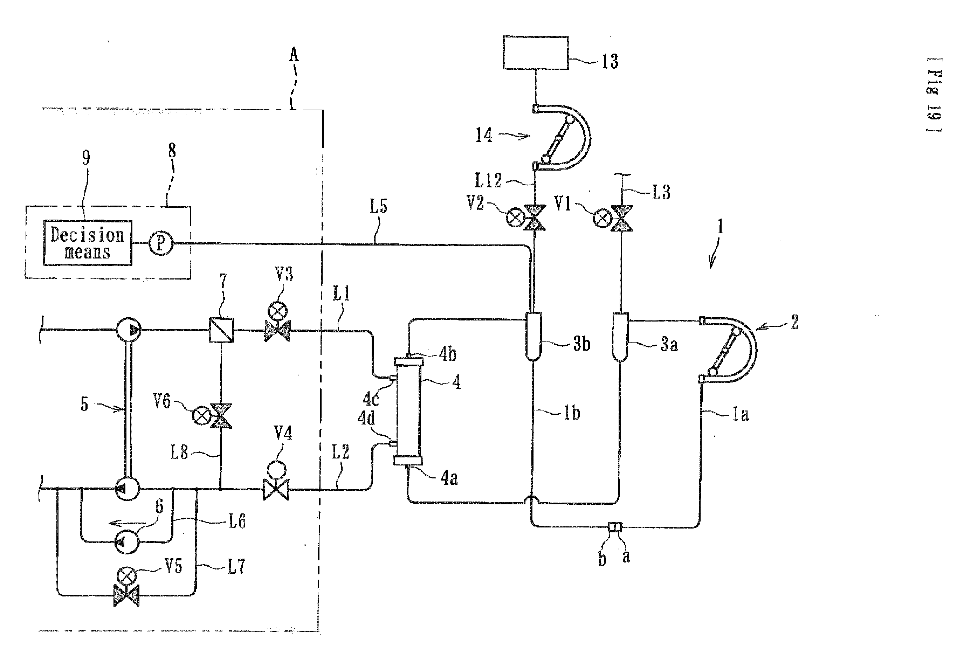

On the other hand, the negative pressure can be applied to the closed circuit as shown in Fig. 19 by forming the sealed closed circuit in the blood circuit 1 with closing the electromagnetic valves V1, V2 to close the overflow line L3 and the physiological saline line L12, and by normally rotating the ultrafiltration pump (liquid supplying means) 6 with closing the electromagnetic valves V3, V5 and V6 and opening other electromagnetic valves to introducing the priming solution (dialysate or physiological saline) in the closed circuit to the dialysate discharging line L2 through the filtration membrane (hollow fiber membrane in this embodiment) of the dialyzer 4.

It is possible to apply the negative pressure to the closed circuit by reversely rotating the substitution infusing pump (liquid supplying means) 14 to the liquid (priming solution) to the physiological saline line L12, or as shown in Fig. 19, it is possible to apply the positive pressure to the closed circuit by reversely rotating the ultrafiltration pump 6 to introduce the liquid (dialysate) to the closed circuit.

Furthermore, according to the present invention, although it is described that both the positive pressure and negative pressure are applied to the closed circuit to perform the liquid leakage inspection, it may be possible to perform either one of the positive pressure application or the negative pressure application. For example, when the liquid pressure variation to the closed circuit is performed by applying the positive pressure to the closed circuit with introducing liquid to the closed circuit from the outside thereof, it is possible to prevent air from being sucked to the blood circuit 1 when liquid leakage would be caused in the negative pressure inspection and thus possible to smoothly and properly perform the blood purification treatment thereafter.

In addition, when the liquid pressure variation relative to the closed circuit is performed by applying the negative pressure to reduce liquid pressure with extracting liquid to the outside of the closed circuit, it is possible to perform the liquid leakage inspection due to the negative pressure of a portion to which the negative pressure is applied (e.g. a portion nearer to the tip end "a" than the blood pump 2 in the arterial blood circuit) in the blood purification treatment and thus possible to perform the liquid leakage inspection based on actions applied during the blood purification treatment.

Furthermore, when the liquid pressure variation relative to the closed circuit is performed by applying the negative pressure to reduce liquid pressure with extracting liquid to the outside of the closed circuit, the liquid leakage detecting means may be formed of an air bubble detecting means being able to detect air bubbles generated in a case of liquid leakage in the blood circuit 1 and a decision means for deciding existence of the liquid leakage in the blood circuit 1 based on detection of bubbles by the air bubble detecting means. In this case, it is possible to use, as a liquid pressure detector for detecting the negative pressure to the closed circuit, an air bubble detector etc. usually connected to a portion nearer the tip end "b" than the air trap chamber 3b in the venous blood circuit 1b in order to reduce the manufacturing cost of the blood purification apparatus. The liquid leakage can be also detected by visual observation based on air bubbles since the air bubbles are contained in liquid.

Although the present invention has been described with reference to several preferable embodiments, it is sufficient if it can detect the liquid leakage at least in the blood circuit and thus it is of course that the present invention can be applied to other liquid circuit (liquid flow path) than the blood circuit. In addition the present invention can be applied to other blood purification apparatus than the hemodialysis apparatus. Furthermore, although it is described that the present invention is applied to the observation apparatus for dialysis (not having dialysate preparing function), it is apparent that the present invention can be applied to the private dialysis apparatus (having dialysate preparing function).

### Applicability in Industries

The present invention can be applied to any other applications having additional functions if they are blood purification apparatus and methods for inspecting liquid leakage adapted to detect liquid leakage in the blood circuit by varying pressure in the closed circuit formed by connecting the tip ends of an arterial blood circuit and a venous blood circuit.

### Explanation of Reference Numerals

| | |
|---|---|
| 1 | blood circuit |
| 1a | arterial blood circuit |
| 1b | venous blood circuit |
| 2 | blood pump |
| 3a | arterial air trap chamber |
| 3b | venous air trap chamber |
| 4 | dialyzer |
| 5 | duplex pump (liquid supplying means) |
| 6 | ultrafiltration pump |
| 7 | filter |
| 8 | liquid leakage detecting means |
| 9 | decision means |
| 10 | substitution infusing port |
| 11 | substitution infusing pump |
| 12 | filter |
| 13 | physiological saline bag |
| 14 | substitution infusing pump |
| P | venous pressure sensor (liquid leakage detecting means, pressure detecting means) |
| L1 | dialysate introducing line |
| L2 | dialysate discharging line |

## Claims

1. A blood purification apparatus comprising:
a blood circuit (1) including an arterial blood circuit (1a) arranged thereon a blood pump (2) and a venous blood circuit (1b) for extracorporeally circulating blood of a patient by the blood pump (2);
a blood purification instrument (4) for purifying the blood of a patient extracorporeally circulated through the blood circuit (1) adapted to be connected with a base end of the arterial blood circuit (1a) and a base end of the venous blood circuit (1b) of the blood circuit (1);
a dialysate introducing line (L1) for introducing dialysate into the blood purifying instrument (4); and
a dialysate discharging line (L2) for discharging the dialysate from the blood purifying instrument (4) **characterized in that** the blood purification apparatus further comprises:
a pressure varying means for varying pressure in a closed circuit under a condition in which the blood circuit (1) is formed as a closed circuit of a sealed condition with connecting the tip end (a) of the arterial blood circuit (1a) and the tip end (b) of the venous blood circuit (1b); and
a liquid leakage detecting means (8) arranged in the closed circuit for detecting liquid leakage in the blood circuit (1) in accordance with pressure variation generated by the pressure varying means.

2. A blood purification apparatus of claim 1 wherein the pressure varying means varies liquid pressure in the closed circuit under a condition in which the blood circuit (1) is filled with priming liquid.

3. A blood purification apparatus of claim 1 or 2 wherein the pressure varying means comprises a liquid supplying means (5) for increasing pressure in the closed circuit by applying a positive pressure to the closed circuit with introducing liquid into the closed circuit from the outside thereof.

4. A blood purification apparatus of claim 3 wherein the blood purification apparatus further comprises a discharging line (L2) for discharging liquid or gas in the closed circuit to the outside thereof; and
wherein the liquid leakage in the blood circuit (1) is detected by the liquid leakage detecting means (8) during a pressurizing step by alternately performing the pressurizing step and a priming step, in the pressurizing step the positive pressure being applied to the closed circuit by introducing liquid into the closed circuit from the outside thereof by the liquid supplying means (5) with keeping a closed condition of the discharging line (L2), and in the priming step, liquid being introduced into the closed circuit from the outside thereof by the liquid supplying means (5) with keeping an opened condition of the discharging line (L2) and then liquid or gas in the closed circuit being discharged from the discharging line (L2).

5. A blood purification apparatus of claim 3 or 4 wherein the liquid leakage detecting means (8) comprises:
a pressure detecting means (P) for detecting pressure in the closed circuit after a positive pressure has been applied to the closed circuit; and
a decision means (9) for deciding existence of liquid leakage in the blood circuit (1) on the basis of pressure detected by the pressure detecting means (P).

6. A blood purification apparatus of claim 1 or 2 wherein the pressure varying means comprises a liquid supplying means (6) for decreasing pressure in the closed circuit by applying a negative pressure to the closed circuit with extracting liquid from the closed circuit to the outside thereof.

7. A blood purification apparatus of claim 6 wherein the liquid leakage detecting means (8) comprises:
a pressure detecting means (P) for detecting pressure in the closed circuit after a negative pressure has been applied to the closed circuit; and
a decision means (9) for deciding existence of liquid leakage in the blood circuit (1) on the basis of the detected pressure by the pressure detecting means (P).

8. A blood purification apparatus of claim 5 or 7 wherein the pressure detecting means (P) comprises one (P1) arranged in the closed circuit and the other one (P2) arranged in the dialysate introducing line (L1) or the dialysate discharging line (L2), and wherein the decision means (9) decides existence of liquid leakage in the blood circuit (1) by comparing a pressure detected by the pressure detecting means (P1) arranged in the closed circuit with a pressure detected by the pressure detecting means (P2) arranged in the dialysate introducing line (L1) or the dialysate discharging line (L2).

9. A blood purification apparatus of claim 6 wherein the liquid leakage detecting means (8) comprises:
an air bubble detecting means for detecting bubbles which would be generated in case of liquid leakage in the blood circuit (1) when the negative pressure is applied to the closed circuit; and
a decision means for deciding existence of liquid leakage on the basis of a fact whether the air bubbles are detected.

10. A blood purification apparatus of any one of claims 3∼9 wherein the liquid supplying means comprises a dialysate pump (5) for introducing the dialysate to the blood purifying instrument (4), a ultrafiltration pump (6) for performing ultrafiltration against blood circulating extracorporeally through the blood circuit (1), or a substitution infusing pump (11) for introducing substitution to the blood circuit (1).

11. A blood purification apparatus of claim 10 wherein the liquid supplying means (5, 6, 11) is a pump being able to perform a normal rotation and a reverse rotation, and wherein either the application of positive pressure or negative pressure to the closed circuit can be achieved by selectively performing the normal rotation or the reverse rotation.

12. A blood purification apparatus of claim 1 or 2 wherein the pressure varying means is able to increase pressure in the closed circuit by applying a positive pressure to the closed circuit with introducing liquid into the closed circuit from the outside thereof and also able to decrease pressure in the closed circuit by applying a negative pressure to the closed circuit with extracting liquid from the closed circuit to the outside thereof, and wherein the liquid leakage detecting means (8) is able to detect liquid leakage both in cases of application of the positive pressure and negative pressure.

13. A blood purification apparatus of claim 12 wherein the positive pressure is applied to the closed circuit after the negative pressure has been applied to the closed circuit.

14. A blood purification apparatus of any one of claims 1∼13 wherein the pressure varying means varies pressure in the closed circuit by introducing liquid to the closed circuit through the dialysate introducing line (L1) or by discharging liquid from the closed circuit to the dialysate discharging line (L2), and wherein the liquid leakage detecting means (8) is able to detect liquid leakage in the dialysate introducing line (L1) or the dialysate discharging line (L2) in addition to liquid leakage in the closed circuit.

15. A method for inspecting liquid leakage of a blood purification apparatus comprising a blood circuit (1) including an arterial blood circuit (1a) arranged thereon a blood pump (2) and a venous blood circuit (1b) for extracorporeally circulating blood of a patient by the blood pump (2); a blood purification instrument (4) for purifying the blood of a patient extracorporeally circulated through the blood circuit (1) adapted to be connected with a base end of the arterial blood circuit (1a) and a base end of the venous blood circuit (1b) of the blood circuit (1); a dialysate introducing line (L1) for introducing dialysate to the blood purifying instrument (4); and a dialysate discharging line (L2) for discharging the dialysate from the blood purifying instrument (4) **characterized in that** the method comprises:
a pressure varying step for varying pressure in a closed circuit under a condition in which the blood circuit (1) is formed as a closed circuit of a sealed condition with connecting the tip end of the arterial blood circuit (1a) and the tip end of the venous blood circuit (1b); and
a liquid leakage detecting step for detecting liquid leakage in the blood circuit (1) in accordance with pressure variation in the pressure varying step.

16. A method for inspecting liquid leakage of a blood purification apparatus of claim 15 wherein in the pressure varying step means, the liquid pressure in the closed circuit is varied under a condition in which the blood circuit (1) is filled with priming liquid.

17. A method for inspecting liquid leakage of a blood purification apparatus of claim 16 wherein in the pressure varying step, the pressure in the closed circuit is increased by applying a positive pressure to the closed circuit with introducing liquid into the closed circuit from the outside thereof.

18. A method for inspecting liquid leakage of a blood purification apparatus of claim 17:
wherein the blood purification apparatus further comprises a discharging line (L2) for discharging liquid or gas in the closed circuit to the outside thereof; and
wherein the liquid leakage detecting step is performed during a pressurizing step by alternately performing the pressurizing step and a priming step, in the pressurizing step the positive pressure being applied to the closed circuit by introducing liquid into the closed circuit from the outside thereof by the liquid supplying means (5) with keeping a closed condition of the discharging line (L2), and in the priming step liquid being introduced into the closed circuit from the outside thereof by the liquid supplying means (5) with keeping a opened condition of the discharging line (L2) and then liquid or gas in the closed circuit being discharged from the discharging line (L2).

19. A method for inspecting liquid leakage of a blood purification apparatus of claim 17 or 18 wherein the liquid leakage detecting step comprises:
a pressure detecting step for detecting pressure in the closed circuit after a positive pressure has been applied to the closed circuit; and
a decision step for deciding existence of liquid leakage in the blood circuit (1) on the basis of the pressure detected in the pressure detecting step.

20. A method for inspecting liquid leakage of a blood purification apparatus of claim 15 or 16 wherein in the pressure varying step, the pressure in the closed circuit is decreased by applying a negative pressure to the closed circuit with extracting liquid from the closed circuit to the outside thereof.

21. A method for inspecting liquid leakage of a blood purification apparatus of claim 20 wherein the liquid leakage detecting step comprises:
a pressure detecting step for detecting pressure in the closed circuit after a negative pressure has been applied to the closed circuit; and
a decision step for deciding existence of liquid leakage in the blood circuit (1) on the basis of pressure detected in the pressure detecting step.

22. A method for inspecting liquid leakage of a blood purification apparatus of claim 19 or 21 wherein in the pressure detecting step, pressures in the closed circuit and in the dialysate introducing line (L1) or the dialysate discharging line (L2) are detected respectively, and wherein the liquid leakage in the blood circuit (1) is decided by comparing a pressure detected in the closed circuit with a pressure detected in the dialysate introducing line (L1) or the dialysate discharging line (L2).

23. A method for inspecting liquid leakage of a blood purification apparatus of claim 20 wherein the liquid leakage detecting step comprises:
an air bubble detecting step for detecting bubbles which would be generated in case of liquid leakage in the blood circuit (1) when the negative pressure is applied to the closed circuit; and
a decision step for deciding existence of liquid leakage on the basis of a fact whether the air bubbles are detected.

24. A method for inspecting liquid leakage of a blood purification apparatus of any one of claims 17∼23 wherein the introduction or discharge of liquid to or from the closed circuit in the pressure varying step is performed by a dialysate pump (5) for introducing the dialysate to the blood purifying instrument (4), a ultrafiltration pump (6) for performing ultrafiltration against blood circulating extracorporeally through the blood circuit (1), or a substitution infusing pump (11) for introducing substitution to the blood circuit (1).

25. A method for inspecting liquid leakage of a blood purification apparatus of claim 24 wherein the introduction or discharge of liquid to or from the closed circuit in the pressure varying step is achieved by a pump being able to perform a normal rotation and a reverse rotation, and wherein either the application of positive pressure or negative pressure to the closed circuit can be achieved by selectively performing the normal rotation or the reverse rotation.

26. A method for inspecting liquid leakage of a blood purification apparatus of claim 15 or 16 wherein in the pressure varying step, the pressure in the closed circuit is increased by applying a positive pressure to the closed circuit with introducing liquid into the closed circuit from the outside thereof and also decreased by applying a negative pressure to the closed circuit with extracting liquid from the closed circuit to the outside thereof, and wherein the liquid leakage detection is performed both in cases of application of the positive pressure and negative pressure.

27. A method for inspecting liquid leakage of a blood purification apparatus of claim 26 wherein the positive pressure is applied to the closed circuit after the negative pressure has been applied to the closed circuit.

28. A method for inspecting liquid leakage of a blood purification apparatus of any one of claims 15∼27 wherein in the pressure varying step, the pressure in the closed circuit is varied by introducing liquid to the closed circuit through the dialysate introducing line (L1) or by discharging liquid from the closed circuit to the dialysate discharging line (L2), and wherein in the liquid leakage detecting step, the liquid leakage in the dialysate introducing line (L1) or the dialysate discharging line (L2) is detected in addition to liquid leakage in the closed circuit.
